# EUROPÄISCHE PATENTSCHRIFT

(11) **EP 0 947 510 B1**
(45) Veröffentlichungstag und Bekanntmachung des Hinweises auf die Patenterteilung: **13.02.2002**
(21) Anmeldenummer: 99106024.5
(22) Anmeldetag: 25.03.1999
(51) Int. Cl.: C07D 249/08

(54) **Verfahren zur Herstellung von 1,3,4-trisubstituierten 1,2,4-Triazoliumsalzen**
Process for the preparation of 1,3,4-trisubstituted 1,2,4-triazolium salts
Procédé de préparation de sels de 1,2,4-triazolium 1,3,4-trisubstitués

(30) Priorität: 02.04.1998 DE 19814801
(43) Veröffentlichungstag der Anmeldung: 06.10.1999
(73) Patentinhaber: BASF AKTIENGESELLSCHAFT, 67056 Ludwigshafen (DE)
(72) Erfinder: Teles, Joaquim Henrique, Dr., 67122 Altrip (DE); Breuer, Klaus, Dr., 67122 Altrip (DE); Enders, Dieter, Prof. Dr., 52074 Aachen-Orsbach (DE); Gielen, Heike, 52072 Aachen (DE)

(56) Entgegenhaltungen:
- EP-A- 0 274 867
- EP-A- 0 526 281
- EP-A- 0 749 965
- CHEMICAL ABSTRACTS, vol. 87, no. 25, 1977 Columbus, Ohio, US; abstract no. 201442, ZIMENKOWSKII B. S. ET AL.: "Certain reactions of alpha, omega-bis(1,3-thiazane-2-thion-4-on-3-yl)a lkanes" XP002107295 & KHIM. GETEROTSIKL. SOEDIN., Nr. 8, 1977, Seiten 1060-1062,
- CHEMICAL ABSTRACTS, vol. 118, no. 19, 1993 Columbus, Ohio, US; abstract no. 191641, AMER M.I.K. ET AL.: "Controlled synthesis of 5-substituted 1-methyl-1H-tetrazoles and 3,5-disubstituted 1,4-dimethyltriazolium salts from N-methylnitrilium trifluoromethanesulfonate salts" XP002107296 & J. CHEM. RES., SYNOP., Nr. 1, 1993, Seiten 4-5,
- M.R. ATKINSON ET AL.: "Synthesis of 1,3-diphenyl-1,2,4-triazole" JOURNAL OF THE AMERICAN CHEMICAL SOCIETY., Bd. 75, 1953, Seite 1471 XP000602283 DC US

## Beschreibung

Die Erfindung betrifft ein neues Verfahren zur Herstellung von 1,3,4-trisubstituierten 1,2,4-Triazoliumsalzen der Formeln Ia bzw. IIa 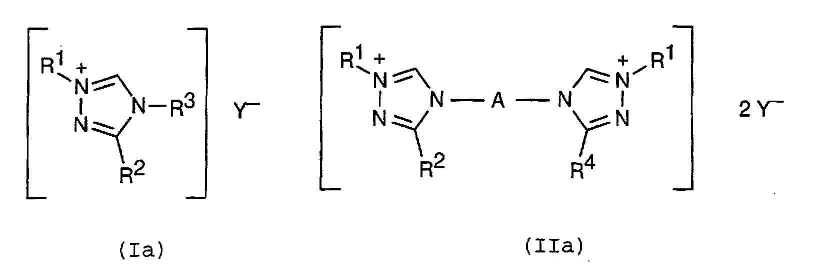 in denen die Substituenten und Variablen unabhängig voneinander
- R¹: C₁₋₃₀-Alkyl, C₃₋₁₂-Cycloalkyl, C₆₋₂₀-Aryl, C₇₋₂₀-Arylalkyl, C₃₋₁₅-Heteroaryl,
- R², R³, R⁴: C₁₋₃₀-Alkyl, C₂₋₃₀-Alkenyl, C₃₋₁₂-Cycloalkyl, C₅₋₁₂-Cycloalkenyl, C₆₋₂₀-Aryl, C₇₋₂₀-Arylalkyl, C₃₋₁₅-Heteroaryl,
oder R² und R³ gemeinsam eine gesättigte oder ungesättigte C₃₋₆-Alkylkette, in der bis zu 2 C-Atome unabhängig voneinander durch -N= (Imino), O, S, NR⁵ oder CHR⁶ substituiert sein können,
oder R² und R⁴ gemeinsam (CH₂)ₐ-E-(CH₂)_{b},
wobei der Rest R¹ Substituenten, ausgewählt aus der Gruppe C₁₋₃₀-Alkyl und/oder C₁₋₃₀-Alkoxy, und die Reste R² bis R⁴ Substituenten, ausgewählt aus der Gruppe Halogene, C₁₋₃₀-Alkyl, C₁₋₃₀-Alkoxy, C₁₋₃₀-Alkanoyloxy, C₆₋₃₀-Aryloxy, C₂₋₂₀-Dialkylamino, Benzoylamino, p-Nitro-benzoylamino und/oder Nitro, tragen können,
- A: (CH₂)_{c}-Bₑ-(CH₂)_{d},
- E: CH₂, CHR⁵, O, S, NR⁶,
- B: CH₂, CHR⁷, O, S, NR⁸,
- R⁵, R⁶, R⁷, R⁸: C₁₋₄-Alkyl, C₇₋₂₀-Alkylaryl, C₇₋₂₀-Arylalkyl,
- a,c: eine ganze Zahl von 1 bis 3,
- b,d: eine ganze Zahl von 1 bis 30,
- e: 0 oder 1,
- Y⁻: ein Anion, ausgewählt aus der Gruppe der Halogenide, Carboxylate der Formeln R⁹CO₂⁻, HCO₂⁻, CF₃CO₂⁻ und p-NO₂-C₆H₄-CO₂⁻, Carbonate der Formel R⁹OCO₂⁻, Sulfate der Formel R⁹OSO₃⁻, Sulfonate der Formeln R⁹SO₃⁻, CF₃SO₃⁻ und p-NO₂-C₆H₄-SO₃⁻, Sulfinate der Formel R⁹SO₂⁻, Imidazolide und Cyanid und
- R⁹: ein aliphatischer, cycloaliphatischer, arylaliphatischer, aromatischer oder alkylaromatischer Rest
bedeuten,
sowie ein neues Verfahren zur Herstellung von 1,3,4-trisubstituierten 1,2,4-Triazoliumsalzen der Formeln Ib bzw. IIb 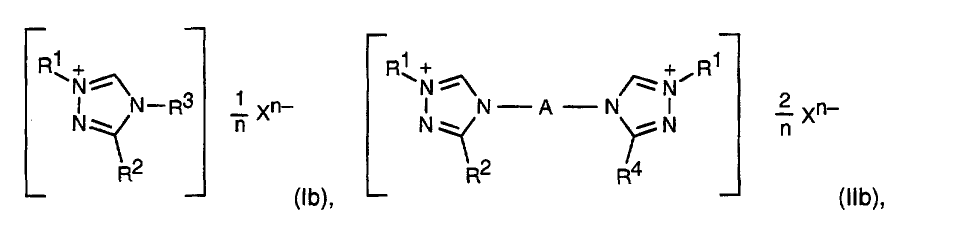 in denen
- Xⁿ⁻: ein Anion mit der Ladungszahl n, ausgewählt aus der Gruppe ClO₄⁻, B(C₆H₅)₄⁻, SbF₆⁻, AsF₆⁻, PF₆⁻, SiF₆²⁻, SO₄²⁻, HSO₄⁻, NO₃⁻, PO₄³⁻, HPO₄²⁻, H₂PO₄⁻, HCO₃⁻, CO₃²⁻,
- n: 1, 2 oder 3
bedeuten und R¹, R², R³, R⁴ und A die eingangs angegebene Bedeutung haben.

Die Erfindung betrifft weiterhin die neuen 1,3,4-trisubstituierten 1,2,4-Triazoliumsalze der Formeln IIa 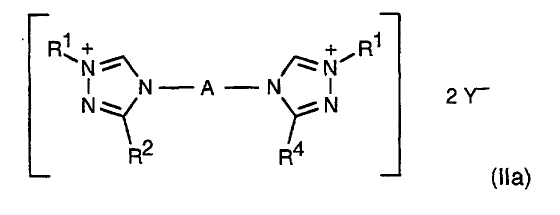 und IIb 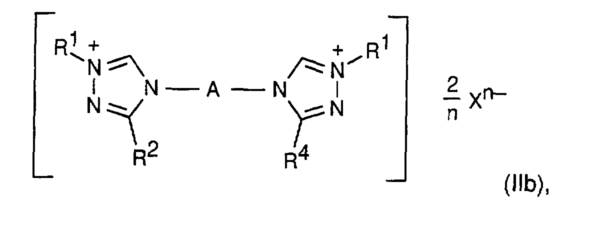 in denen die Substituenten und Variablen die oben angegebene Bedeutung haben, und die neuen Bis-imidoylverbindungen der Formel IV 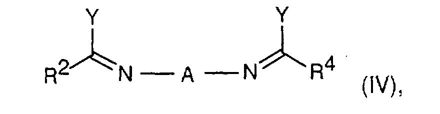 die bei der erfindungsgemäßen Herstellung der 1,2,4-Triazoliumsalze IIa und IIb als Zwischenprodukte auftreten.

1,2,4-Triazoliumsalze haben große technische Bedeutung als *ex*-oder *in situ*-Vorläufer für entsprechende nukleophile Carbene, die als sehr gute Katalysatoren für Acyloin-Kondensationen [J. H. Teles et al., Helv. Chim. Acta 79, 61-83 (1996); EP-A-587 044], Stetter-Reaktionen [D. Enders et al., Helv. Chim. Acta 79, 1899-1902 (1996)] oder als Liganden für Übergangsmetalle [M. Regitz, Angew. Chem. 108, Seite 791ff (1996); Angew. Chem. Int. Ed. Engl. 35, Seite 725ff (1996)] bekannt sind.

Zur Herstellung von substituierten 1,2,4-Triazoliumsalzen sind verschiedene Synthesewege bekannt.

In R. Walentowski et al., Z. Naturforsch. B, 25, Seite 1421-3 (1970) und H.G.O. Becker et al., J. Prakt. Chem., 330, Seite 325-37 (1988) wird die Synthese von 1,3,4-trisubstituierten 1,2,4-Triazoliumsalzen ausgehend von den entsprechenden 1,3,4-trisubstituierten 1,2,4-Triazolthionen-(5) durch oxidative Entschwefelung mit Salpetersäure oder Wasserstoffperoxid beschrieben.

T. Eicher, S. Hünig et al., Chem. Ber. 102, 3159-75 (1969) und GB-A-1,118,426 beschreiben die Synthese von 1,2,4-Triazoliumsalzen durch Umsetzung von Iminen oder aromatischen N-Heterocyclen vom Pyridintyp mit Alkoxydiazeniumsalzen.

Die Synthese von 1,2,4-Triazoliumsalzen durch Umsetzung von 1,3,4-Oxadiazoliumsalzen mit primären Aminen ist aus G.V. Boyd, J. Chem. Soc. C, Seite 409-14 (1971) bekannt.

In DD-A-257 312 wird die Synthese von 1,4-disubstituierten 3-Mercapto-1,2,4-triazoliumsalzen durch Umsetzung von Thiosemicarbaziden mit Ameisensäure und die Synthese von 1,4-Diphenyl-3-anilino-1,2,4-triazoliumhydroxid durch Umsetzung von Triphenylaminoguanidin mit Formalin und FeCl₃-Lösung beschrieben.

Diese Methoden sind jedoch verhältnismäßig kompliziert, eignen sich daher nur bedingt für die Übertragung in den technischen Maßstab und haben insoweit den Nachteil, daß sie jeweils nur in bestimmten Fällen mit befriedigendem Erfolg anwendbar sind.

EP-A-749 965 offenbart ein Verfahren zur Herstellung von 1,2,4-Triazoliumsalzen indem man ein Amidrazon mit einer Carbonsäure oder einem funktionellen Derivat dieser Säure umsetzt.

Der Erfindung lag daher die Aufgabe zugrunde, den genannten Nachteilen durch das Auffinden von einfachen, universell geeigneten Syntheseverfahren zur Herstellung von 1,3,4-trisubstituierten 1,2,4-Triazoliumsalzen der Formeln Ia, Ib bzw. IIa, IIb abzuhelfen. Außerdem war es eine Aufgabe, neue 1,3,4-trisubstituierte 1,2,4-Triazoliumsalze IIa und IIb aufzufinden, die sich vorteilhaft als Vorläufer für Katalysatoren oder Übergangsmetall-Liganden verwenden lassen.

Demgemäß wurde ein Verfahren zur Herstellung von 1,3,4-trisubstituierten 1,2,4-Triazoliumsalzen der Formeln Ia bzw. IIa 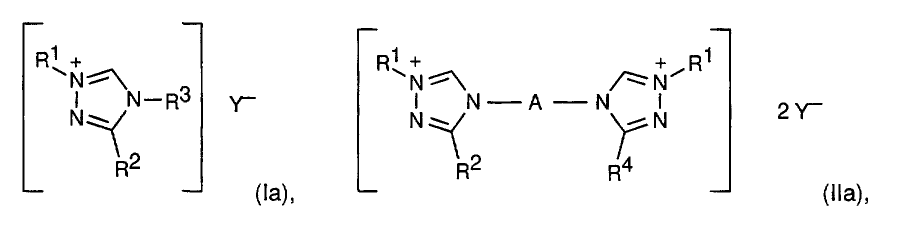 gefunden,
welches dadurch gekennzeichnet ist, daß man eine Imidoylverbindung der Formel III bzw. eine Bis-imidoylverbindung der Formel IV 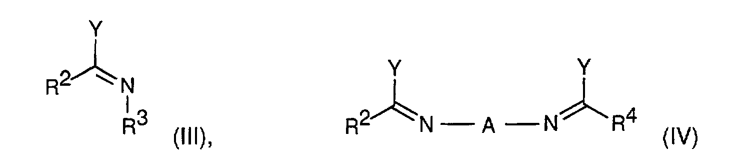 mit einem N-Formyl-hydrazin der Formel V 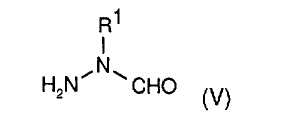 bei Temperaturen von -78 bis 130°C umsetzt.

Es wurde weiterhin ein Verfahren zur Herstellung von 1,3,4-trisubstituierten 1,2,4-Triazoliumsalzen der Formeln Ib bzw. IIb 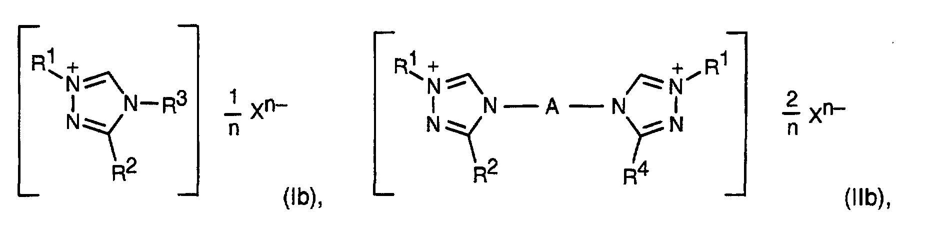 gefunden,
welches dadurch gekennzeichnet ist, daß man 1,2,4-Triazoliumsalze der Formeln Ia bzw. IIa nach dem erfindungsgemäßen Verfahren herstellt und anschließend mit einer Verbindung der Formel MₙXₘ,
wobei M für Wasserstoff mit der Oxidationszahl m = 1 oder für ein Metall der ersten oder zweiten Gruppe des Periodensystems mit der Oxidationszahl m steht, umsetzt.

Schließlich wurden die neuen 1,3,4-trisubstituierten 1,2,4-Triazoliumsalze der eingangs definierten Formeln IIa 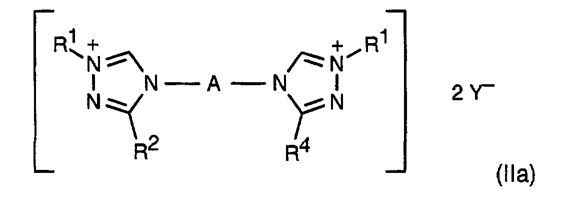 und IIb 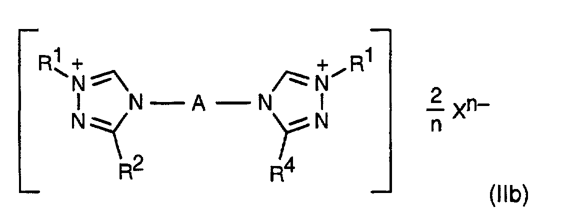 sowie die, bei der erfindungsgemäßen Herstellung der 1,2,4-Triazoliumsalze IIa und IIb als Zwischenprodukte auftretenden, neuen Bis-imidoylverbindungen der Formel IV 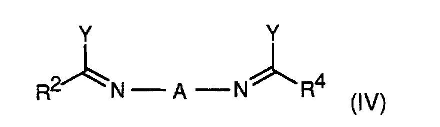 gefunden.

Die Substituenten R¹ bis R⁹ sowie die Bezeichnungen X, Y, Z, A, E, B, a, b, c, d, e und n in den Verbindungen der Formeln Ia, Ib, IIa, IIb, III, IV und V haben unabhängig voneinander folgende Bedeutungen:
- R¹: - C₁₋₃₀-Alkyl, vorzugsweise C₁₋₂₀-Alkyl, bevorzugt C₁₋₁₃-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, 2-Ethyl-hexyl, n-Nonyl, iso-Nonyl, n-Decyl, iso-Decyl, n-Undecyl, n-Dodecyl, iso-Dodecyl, besonders bevorzugt C₁₋₄-Alkyl, wie Methyl, Ethyl, n-Propyl, iso-Propyl, n-Butyl, iso-Butyl, sec.-Butyl und tert.-Butyl,
- C₃₋₁₂-Cycloalkyl, vorzugsweise C₃₋₈-Cycloalkyl, wie Cyclopropyl, Cyclobutyl, Cyclopentyl, Cyclohexyl, Cycloheptyl und Cyclooctyl, bevorzugt Cyclopentyl, Cyclohexyl und Cyclooctyl, besonders bevorzugt Cyclopentyl und Cyclohexyl,
- C₆₋₂₀-Aryl, wie Phenyl, 1-Naphthyl, 2-Naphthyl, 1-Anthryl, 2-Anthryl, 9-Anthryl, bevorzugt Phenyl, 1-Naphthyl und 2-Naphthyl, besonders bevorzugt Phenyl,
- C₇₋₂₀-Arylalkyl, wie Benzyl, 1-Phenethyl, 2-Phenethyl, 1-Naphthylmethyl, 2-Naphthylmethyl, Phenanthrylmethyle, 4-tert.-Butyl-phenyl-methyl, 1-Phenylpropyl, 2-Phenylpropyl, 3-Phenylpropyl, 1-Phenylbutyl, 2-Phenylbutyl, 3-Phenylbutyl und 4-Phenylbutyl, besonders bevorzugt Benzyl, 1-Phenethyl und 2-Phenethyl,
- C₃₋₁₅-Heteroaryl, wie 2-Pyridinyl, 3-Pyridinyl, 4-Pyridinyl, Chinolinyl, Pyrazinyl, Pyrrol-3-yl, Thienyl, Imidazol-2-yl, 2-Furanyl und 3-Furanyl,
- R², R⁴: - C₁₋₃₀-Alkyl, wie für R¹ definiert,
- C₂₋₃₀-Alkenyl, vorzugsweise C₂₋₂₀-Alkenyl, bevorzugt C₂₋₁₂-Alkenyl, wie Ethenyl, 2-Propen-1-yl, 2-Propen-2-yl, 2-Buten-1-yl, 2-Buten-2-yl, 3-Buten-1-yl, 3-Buten-2-yl, 2-Penten-1-yl, 4-Penten-1-yl, 2-Hexen-1-yl, 5-Hexen-1-yl,
- C₃₋₁₂-Cycloalkyl, wie für R¹ definiert,
- C₅₋₁₂-Cycloalkenyl, vorzugsweise C₅₋₈-Cycloalkenyl, wie Cyclopenten-1-yl, Cyclopenten-3-yl, Cyclohexen-1-yl, Cyclohexen-3-yl, Cyclohexen-4-yl, Cyclohepten-1-yl und Cycloocten-1-yl,
- C₆₋₂₀-Aryl, wie Phenyl, p-NO₂-Phenyl, p-CH₃O-Phenyl, p-Cl-Phenyl, 1-Naphthyl, 2-Naphthyl, 1-Anthryl, 2-Anthryl, 9-Anthryl bevorzugt Phenyl, 1-Naphthyl, 2-Naphthyl, besonders bevorzugt Phenyl und p-NO₂-Phenyl,
- C₇₋₂₀-Arylalkyl, wie für R¹ definiert,
- C₃₋₁₅-Heteroaryl, wie für R¹ definiert,
- oder R² und R⁴ gemeinsam eine Kette der Formel (CH₂)ₐ-E-(CH₂)_{b}, mit den Bedeutungen für E, a und b wie unten definiert,
   wie zum Beispiel -(CH₂)₃-, -(CH₂)₄-, -(CH₂)₆, -(CH₂)₈, -(CH₂)₂-O-(CH₂)₂-, -(CH₂)₃-O-(CH₂)₃-, -(CH₂)₂-N(CH₃)-(CH₂)₂-, -(CH₂)₂-N(C₆H₅)-(CH₂)₂-, -(CH₂)₂-N(CH₂C₆H₅)-(CH₂)₂-,
- oder R² und R³ gemeinsam eine gesättigte oder ungesättigte C₃₋₆-Alkylkette, wie unten bei R³ definiert,
- R³: - C₁₋₃₀-Alkyl, wie für R¹ definiert,
- C₂₋₃₀-Alkenyl, wie für R² und R⁴ definiert,
- C₃₋₁₂-Cycloalkyl, vorzugsweise C₃₋₈-Cycloalkyl, wie Cyclopropyl, Cyclobutyl, Cyclopentyl, Cyclohexyl, 2-(Benzoylamino)-substituiertes Cyclohexyl, 2-(p-Nitrobenzoylamino)-substituiertes Cyclohexyl, Cycloheptyl und Cyclooctyl, bevorzugt Cyclopentyl, Cyclohexyl und 2-(p-Nitrobenzoylamino)-substituiertes Cyclohexyl,
- C₅₋₁₂-Cycloalkenyl, wie für R² und R⁴ definiert,
- C₆₋₂₀-Aryl, wie für R¹ definiert,
- C₇₋₂₀-Arylalkyl, wie Benzyl, 1-Phenethyl, 2-Phenethyl, 1-Naphthylmethyl, 2-Naphthylmethyl, Phenanthrylmethyle, 4-tert.-Butyl-phenyl-methyl, 1-Phenylpropyl, 2-Phenylpropyl, 3-Phenylpropyl, 1-Phenylbutyl, 2-Phenylbutyl, 3-Phenylbutyl, 4-Phenylbutyl und 1-Phenyl-1-acetoxy-propan-2-yl, besonders bevorzugt Benzyl, (R)-1-Phenethyl, (S)-1-Phenethyl, (1S, 2R)-1-Phenyl-l-acetoxy-propan-2-yl und (1R, 2S)-1-Phenyl-1-acetoxy-propan-2-yl,
- C₃₋₁₅-Heteroaryl, wie für R¹ definiert,
- oder R² und R³ gemeinsam eine gesättigte oder ungesättigte C₃₋₆-Alkylkette, in der bis zu 2 C-Atome unabhängig voneinander durch -N= (Imino), O, S, NR⁵ oder CHR⁶ substituiert sein können, mit den Bedeutungen für R⁵ und R⁶ wie unten angegeben, wie beispielsweise -(CH₂)₃-, -(CH₂)₄-, -(CH₂)₅-, -(CH₂)₆-, -CH₂-CH=CH-, -S-CH=CH-, -O-CH=CH-, -CH=CH-CH=CH-, -CH=CH-CH₂-CH₂-, -CH=N-CH=CH-,-CH=CH-N=CH-, -CH=CH-CH=N-, -(CH₂)₂-O-CH₂-, -(CH₂)₂-O-(CH₂)₂-, -CH₂-N(CH₃)-CH₂-, -CHCH₃-(CH₂)₂-, -CHCH₃-(CH₂)₃-, besonders bevorzugt -(CH₂)₃-,-(CH₂)₄-,-(CH₂)₅-, -CH=CH-CH=CH-, -CH=N-CH=CH-, -CH=CH-N=CH- und -CH=CH-CH=N-,
- R⁵, R⁶, R⁷, R⁸: - C₁₋₄-Alkyl, wie Methyl, Ethyl, n-Propyl, iso-Propyl, n-Butyl, iso-Butyl, sec.-Butyl, tert.-Butyl, bevorzugt Methyl, Ethyl und tert.-Butyl,
- C₇₋₂₀-Alkylaryl, bevorzugt C₇₋₁₂-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-iso-Propylphenyl, 3-iso-Propylphenyl, 4-iso-Propylphenyl und 4-tert.-Butylphenyl,
- C₇₋₂₀-Arylalkyl, wie für R¹ definiert,
- R⁹: - ein aliphatischer, cycloaliphatischer, arylaliphatischer, aromatischer oder alkylaromatischer Rest, wie zum Beispiel C₁₋₃₀-Alkyl, C₃₋₁₂-Cycloalkyl, C₇₋₂₀-Arylalkyl, C₆₋₂₀-Aryl, jeweils wie oben für R¹ definiert, und C₇₋₂₀-Alkylaryl, wie oben für R⁵ bis R⁸ definiert,
- A: - eine Kette der Formel (CH₂)_{c}-Bₑ-(CH₂)_{d}, mit Bedeutungen für B, c, d und e wie unten angegeben, wie beispielsweise -(CH₂)₂-, -(CH₂)₃-, -(CH₂)₄-, -(CH₂)₅-, - (CH₂)₆-, -(CH₂)₈, -(CH₂)₂-O-(CH₂)₂-, -(CH₂)₂-S-(CH₂)₂-, -(CH₂)₃-O-(CH₂)₃-, -(CH₂)₂-N(CH₃)-(CH₂)₂-, besonders bevorzugt -(CH₂)₂- und -(CH₂)₆-,
- E: - CH₂, CHR⁵, O, S, NR⁶, bevorzugt CH₂, O, NR⁶,
- B: - CH₂, CHR⁷, O, S, NR⁸, bevorzugt CH₂, O, NR⁸,
- a, c: - eine ganze Zahl von 1 bis 3, beispielsweise 1 oder 2,
- b, d: - eine ganze Zahl von 1 bis 30, beispielsweise 1, 2, 3, 4, 8, 10, 12,
- e: - 0 oder 1,
- Y⁻: ein Anion mit der Ladungszahl 1, ausgewählt aus der Gruppe
- der Halogenide, Carboxylate der Formel R⁹CO₂⁻, Carbonate der Formel R⁹OCO₂⁻, Sulfate der Formel R⁹OSO₃⁻, Sulfonate der Formel R⁹SO₃⁻, Sulfinate der Formel R⁹SO₂⁻, Imidazolide, Cyanid,
bevorzugt F-, Cl⁻, Br⁻, I⁻, CH₃OCO₂⁻, C₂H₅OCO₂⁻, C₆H₅CH₂OCO₂⁻, HCO₂⁻, CH₃CO₂⁻, C₂H₅CO₂⁻, CF₃CO₂⁻, P-NO₂-C₆H₄-CO₂⁻, CH₃OSO₃⁻, CH₃SO₃⁻, CF₃SO₃⁻, C₆H₅-SO₃⁻, P-CH₃-C₆H₄-SO₃⁻, p-NO₂-C₆H₄-SO₃⁻, NC⁻, Imidazol-1-yl,
besonders bevorzugt Cl⁻,
- xⁿ⁻: ein Anion mit den Ladungszahlen n = 1, 2 oder 3, ausgewählt aus der Gruppe
- ClO₄⁻, B(C₆H₅)₄⁻, SbF₆⁻, AsF₆⁻, PF₆⁻, SiF₆²⁻, SO₄²⁻, HSO₄⁻, NO₃⁻, PO₄³⁻. HPO₄²⁻, H₂PO₄⁻, HCO₃⁻, CO₃²⁻,
bevorzugt ClO₄⁻ und B(C₆H₅)₄⁻.

Der Rest R¹ kann gegebenenfalls substituiert sein, z. B. durch C₁₋₃₀-Alkyl und/oder C₁₋₃₀-Alkoxy.

Die Reste R², R³ und R⁴ können gegebenenfalls substituiert sein, z. B. durch Halogene, C₁₋₃₀-Alkyl, C₁₋₃₀-Alkoxy, C₁₋₃₀-Alkanoyloxy, C₆₋₃₀-Aryloxy, C₂₋₂₀-Dialkylamino, Benzoylamino, p-Nitro-benzoylamino und/oder Nitro.

Dabei kann die Anzahl dieser Substituenten in R¹ bis R⁴ in Abhängigkeit von der Art des Restes 0 bis 5, vorzugsweise 0 bis 3, insbesondere 0, 1 oder 2 betragen. Als Substituenten kommen in Betracht:
- Halogene, wie F, Cl, Br, I,
- C₁₋₃₀-Alkyl, wie oben für R¹ definiert,
- C₁₋₃₀-Alkoxy, bevorzugt C₁₋₈-Alkoxy, wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy, besonders bevorzugt C₁₋₄-Alkoxy, wie Methoxy, Ethoxy, n-Propoxy, iso-Propoxy, n-Butoxy, iso-Butoxy, sec.-Butoxy und tert.-Butoxy,
- C₁₋₃₀-Alkanoyloxy, bevorzugt C₁₋₈-Alkanoyloxy, wie Formyloxy, Acetyloxy, Propanoyloxy, Butanoyloxy, t-Butylcarbonyloxy, besonders bevorzugt Acetyloxy,
- C₆₋₃₀-Aryloxy, wie Phenoxy, 1-Naphthoxy und 2-Naphthoxy, bevorzugt Phenoxy,
- C₂₋₂₀-Dialkylamino, bevorzugt C₂₋₁₂-Dialkylamino, besonders C₂₋₈-Dialkylamino, wie N,N-Dimethylamino, N,N-Diethylamino, N,N-Dipropylamino, N,N-Di-(1-methylethyl)amino, N,N-Dibutylamino, N,N-Di-(1-methylpropyl)-amino, N,N-Di-(2-methylpropyl)amino, N,N-Di-(1,1-dimethylethyl)-amino, N-Ethyl-N-methylamino, N-Methyl-N-propylamino, N-Methyl-N-(1-methylethyl)-amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methyl-ethyl)-amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methyl-propyl)-N-propylamino, N-(1-Methylpropyl)-N-propylamino, N-(2-Methyl-propyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(1-methpropyl)amino, N-(1-Methylethyl)-N-(2-methylpro-pyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino, Diallylamino, Dicyclohexylamino,
- Benzoylamino: (C₆H₅)-C (O) -NH-
- p-Nitro-benzoylamino: p-NO₂- (C₆H₄) -C (O) -NH-
- Nitro: -NO₂.

### Die Verfahren lassen sich wie folgt ausführen:

Zur Herstellung eines 1,3,4-trisubstituierten 1,2,4-Triazoliumsalzes der Formel Ia wird eine Imidoylverbindung der Formel III mit einem N-Formyl-hydrazin der Formel V unter Freisetzung eines Moläquivalents Wasser umgesetzt.

Analog wird zur Herstellung eines 1,3,4-trisubstituierten 1,2,4-Triazoliumsalzes der Formel IIa eine Bis-imidoylverbindung der Formel IV mit einem N-Formyl-hydrazin der Formel V unter Freisetzung von zwei Moläquivalenten Wasser umgesetzt.

Diese erfindungsgemäßen Umsetzungen werden durch die beiden folgenden Reaktionsschemen veranschaulicht, wobei, wie ersichtlich, beiden Reaktionsschemen dasselbe Reaktionsprinzip zugrunde liegt.

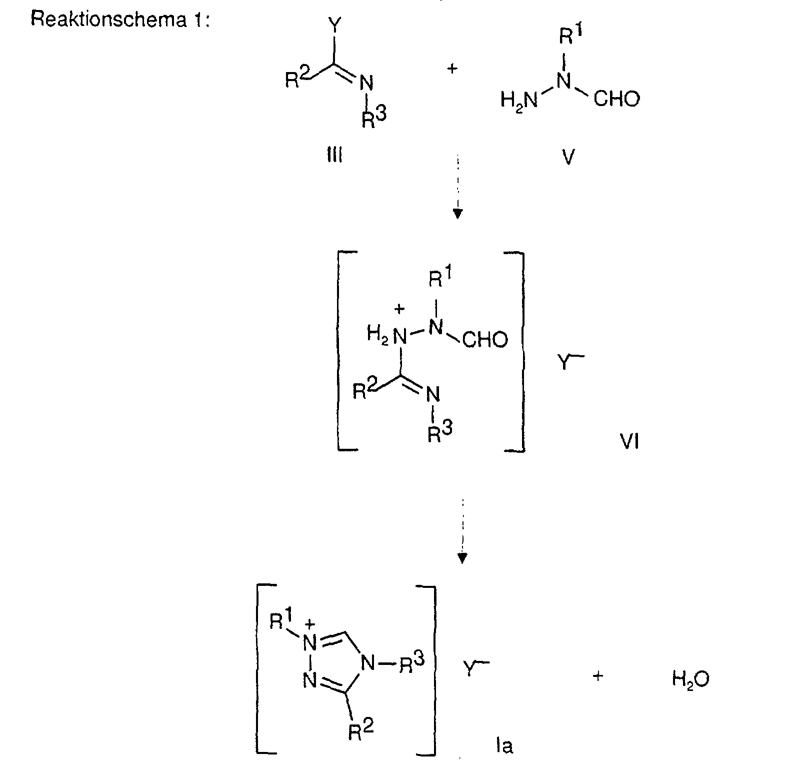

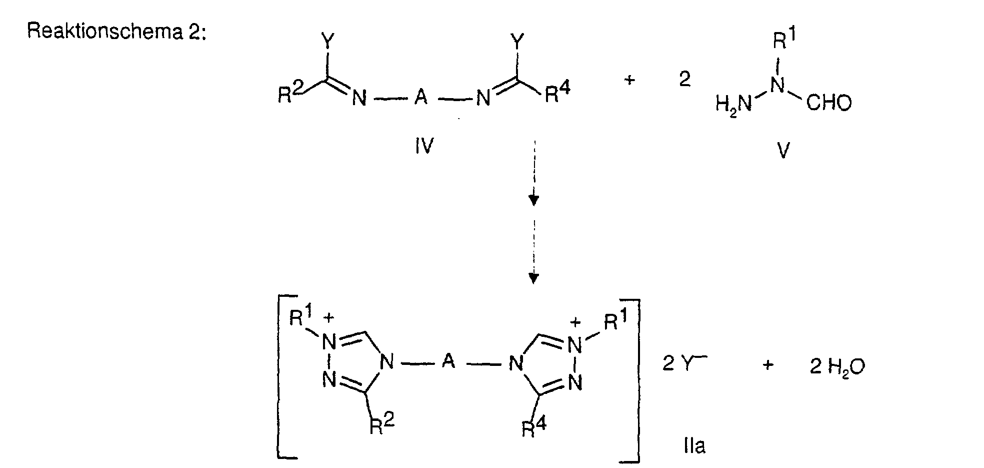

Die Umsetzung zu den 1,2,4-Triazoliumsalzen Ia bzw. IIa erfolgt im allgemeinen in flüssiger Phase, wobei gewöhnlich die Imidoylverbindung III bzw. IV, gegebenenfalls in einen inerten Lösungsmittel gelöst, vorgelegt und mit dem N-Formyl-hydrazin V versetzt wird.

Gegebenenfalls während dieser Reaktionen gebildete Zwischenstufen, wie zum Beispiel das durch nukleophile Substitution der Abgangsgruppe Y gebildete Ammoniumsalz VI (Reaktionsschema 1), werden in der Regel nicht isoliert, sondern direkt weiter zum 1,2,4-Triazoliumsalz Ia (bzw. analog: IIa) umgesetzt.

Um die in der Regel *in situ* erfolgende Wasserabspaltung unter Bildung der 1,2,4-Triazoliumringe von Ia bzw. IIa zu beschleunigen, können der Reaktionsmischung, bevorzugt nach beendeter Zugabe des N-Formyl-hydrazins V, Verbindungen, die die Wasserabspaltung begünstigen, wie zum Beispiel Carbonsäureanhydride oder Carbodiimide zugesetzt werden. Die Menge dieser Zusätze liegt im allgemeinen bei 0,5 bis 100 Moläquivalenten, bevorzugt 1 bis 10 Moläquivalenten, bezogen auf die Molmenge an Reaktionswasser. Beispiele für solche Verbindungen sind Acetanhydrid, Propionsäureanhydrid, Formylacetat oder N,N'-Dicyclohexylcarbodiimid. Bevorzugt ist Acetanhydrid.

Zum gleichen Zweck können der Reaktionsmischung auch wasseranziehende Reagenzien, wie zum Beispiel Natriumsulfat, Magnesiumsulfat oder Zeolithe, zugesetzt werden. Diese Reagenzien werden im allgemeinen in Mengen von 1 bis 20 Gewichtsäquivalenten, bezogen auf das Reaktionswasser, verwendet.

Für gleichen Zweck sind auch Lösungsmittel, die in der Siedehitze als Schleppmittel für Wasser fungieren, wie zum Beispiel Toluol oder Cyclohexan, einsetzbar.

Die Reaktionszeiten liegen gewöhnlich im Bereich von einer Stunde bis fünf Tagen und lassen sich in der Regel verkürzen, wenn während der Reaktion eine Base in Mengen von zum Beispiel 0,2 bis 20 Moläquivalenten bezogen auf die eingesetzte Imidoylverbindung, zugegen ist.

Geeignete Basen sind beispielsweise tertiäre Amine, wie Triethylamin, Tributylamin, Ethyldiisopropylamin, N,N-Dimethylanilin, 4-Dimethylamino-pyridin, oder aromatische N-Heterocyclen, wie Pyridin oder Chinolin.

Die Reaktion der Imidoylverbindung III bzw. IV mit dem N-Formyl-hydrazin V verläuft in vielen Fällen exotherm. Die Umsetzung wird im allgemeinen zunächst bei Temperaturen von -78 bis + 50 °C, bevorzugt -40 bis +25 °C, begonnen und bei Temperaturen von +25 bis +130 °C, bevorzugt +25 bis +70 °C, zu Ende geführt.

Bevorzugte Lösungsmittel für diese Reaktion sind inerte organische aprotische Lösungsmittel, wie zum Beispiel Methylenchlorid, 1,2-Dichlorethan, Toluol, Xylole, Ether, wie Diethylether, Tetrahydrofuran, Methyl-tert.-butyl-ether oder 1,4-Dioxan, Ester, wie Essigsäureethylester, oder Gemische hiervon, bevorzugt Methylenchlorid.

Die Umsetzung kann diskontinuierlich oder kontinuierlich, drucklos oder unter Druck, bevorzugt diskontinuierlich und bei Normaldruck, durchgeführt werden.

Als Reaktionsbehälter kommen gängige Reaktoren, wie zum Beispiel Rührkessel oder Rührbehälterkaskaden, in Betracht.

Für die Herstellung der 1,2,4-Triazoliumsalze Ia werden gewöhnlicherweise pro Mol Imidoylverbindung III 0,5 bis 5 Mol, bevorzugt 1 bis 2,5 Mol, des N-Formyl-hydrazins V eingesetzt.

Für die Herstellung der 1,2,4-Triazoliumsalze IIa werden gewöhnlicherweise pro Mol Imidoylverbindung IV 1 bis 10 Mol, bevorzugt 2 bis 5 Mol, des N-Formyl-hydrazins V eingesetzt.

Praktisch alle Imidoylverbindungen III bzw. IV, in denen der Substituent Y eine durch eine Aminogruppe substituierbare Abgangsgruppe darstellt, sind erfindungsgemäß einsetzbar.

Auch α-Y substituierte aromatische oder aliphatische Heterocyclen, die das Imidoyl-Strukturelement enthalten, wie z. B. 2-Chlorpyridin oder l-Aza-2-chlor-1-cyclopenten, sind einsetzbar.

Als Bis-imidoylverbindungen IV sind auch solche einsetzbar, in denen die Reste R² und/oder R⁴ zusammen mit der Kette A einen 5-bis 8-gliedrigen Ring bilden, wie z.B. in Bis-(1-aza-2-chlor-1-cyclopenten-5-yl)-methan.

### Beispiele für Imidoylverbindungen III sind:

N-Phenyl-benzimidoylchlorid, N-Phenyl-p-nitro-benzimidoylchlorid, N-Methyl-benzimidoylchlorid, N-Methyl-benzimidoylbromid, N-Methyl-benzimidoylacetat, N-Methyl-benzimidoylmethylsulfat, N-Methyl-p-nitro-benzimidoylchlorid, N-Benzyl-benzimidoylchlorid, N-(p-Tolyl)-isobutyrimidoylchlorid, N-Methyl-ethanimidoylchlorid, N-((R,S)-1-phenylethyl)-ethanimidoylchlorid, N-((R)-1-phenylethyl)-ethanimidoylchlorid, N-((S)-1-phenylethyl)-ethanimidoylchlorid, N-(n-Butyl)-2-ethylhexanimidoylchlorid, N-Phenyl-trichloroacetimidoylchlorid, N-(1-Phenyl-l-acetoxy-propan-2-yl)-ethanimidoylchlorid, N-((1R, 2S)-1-Phenyl-l-acetoxy-propan-2-yl)-ethanimidoylchlorid, N-(2-(p-Nitrobenzoylamino)-cyclohexyl)-p-nitro-benzimidoylchlorid, 1-Aza-2-chlor-1-cyclopenten, l-Aza-2-chlor-1-cyclohexen, 1-Aza-2-chlor-1-cyclohepten, 2-Chlorpyridin, 2-Chlorpyrazin, 4-Chlorpyrimidin, 3-Chlorpyridazin, 3-Chlor-1,4-oxazin.

Bevorzugt sind Imidoylverbindungen mit bis zu 40 C-Atomen, besonders bevorzugt sind Imidoylchloride mit bis zu 40 C-Atomen, insbesondere N-Methyl-ethanimidoylchlorid, N-(1-phenylethyl)-ethanimidoylchlorid, N-Methyl-benzimidoylchlorid, N-(trans-2-(p-Nitrobenzoylamino)-cyclohexyl)-p-nitro-benzimidoylchlorid, 2-Chlorpyridin und 2-Chlorpyrazin.

### Beispiele für Bis-imidoylverbindungen IV sind:

1,10-Diphenyl-1,10-dichlor-2,9-diaza-deca-1,9-dien, 1,10-Diphenyl-1,10-dibrom-2,9-diaza-deca-1,9-dien, 1,10-Diphenyl-1,10-diacetoxy-2,9-diaza-deca-1,9-dien, 1,8-Diphenyl-1,8-dichlor-2,7-diaza-octa-1,7-dien, 1,7-Diphenyl-1,7-dichlor-2,6-diazahepta-1,6-dien, 1,6-Diphenyl-1,6-dichlor-2,5-diaza-hexa-1,5-dien, Bis-(1-aza-2-chlor-1-cyclopenten-5-yl)-methan, 1,2-Bis-(1-aza-2-chlor-1-cyclopenten-5-yl)-ethan, Bis-(1-aza-2-chlor-1-cyclohexen-6-yl)-methan, 1,2-Bis-(1-aza-2-chlor-1-cyclohexen-6-yl)-ethan.

Bevorzugt sind Bis-imidoylverbindungen mit bis zu 40 C-Atomen, besonders bevorzugt sind Bis-imidoylchloride mit bis zu 40 C-Atomen, insbesondere aus wirtschaftlichen Gründen 1,10-Diphenyl-1,10-dichlor-2,9-diaza-deca-1,9-dien.

Bezüglich der erfindungsgemäß einsetzbaren 1-substituierten 1-Formyl-hydrazine V sind keine Einschränkungen bekannt. Beispiele für 1-Formyl-hydrazine V sind:

1-Formyl-l-methyl-hydrazin, l-Formyl-l-ethyl-hydrazin, 1-Formyl-1-n-propyl-hydrazin, l-Formyl-l-isopropyl-hydrazin, 1-Formyl-1-n-butyl-hydrazin, 1-Formyl-l-cyclopentyl-hydrazin, 1-Formyl-1-cyclohexyl-hydrazin, 1-Formyl-l-phenyl-hydrazin, 1-Formyl-1-(1-phenethyl)-hydrazin, 1-Formyl-1-benzyl-hydrazin.

Bevorzugt sind N-Formyl-hydrazine mit bis zu 20 C-Atomen. Besonders bevorzugt ist aus wirtschaftlichen Gründen 1-Formyl-1-methyl-hydrazin.

Die Reinigung und Isolierung der erhaltenen 1,2,4-Triazoliumsalze Ia und IIa erfolgt mit den üblichen Methoden und Verfahren, wie zum Beispiel Kristallisation, Umkristallisation und Säulenchromatographie unter Verwendung von Lösungsmitteln wie zum Beispiel Diethylether, Methyl-tert.-butyl-ether, Tetrahydrofuran, Methanol, Ethanol, Aceton, Diethylketon, Toluol, Methylenchlorid, 1,2-Dichlorethan, Essigsäureethylester, oder Gemischen hiervon.

Zur Synthese der 1,2,4-Triazoliumsalze Ib bzw. IIb werden die nach den obigen Verfahren hergestellten 1,2,4-Triazoliumsalze Ia bzw. IIa mit einer Verbindung der Formel MₙXₘ entsprechend den folgenden Gleichungen umgesetzt.

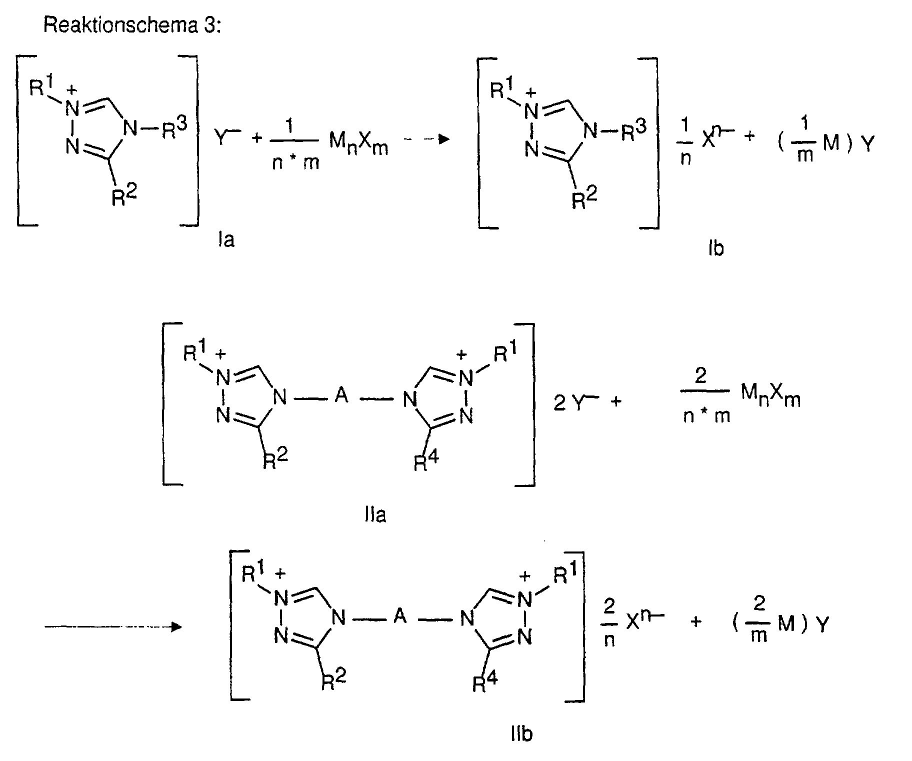

In den Verbindungen der Formel MₙXₘ steht M für Wasserstoff mit der Oxidationszahl m = 1 oder für ein Metall der ersten oder zweiten Gruppe des Periodensystems mit der Oxidationszahl m = 1 bzw. 2.

Beispiele für Metalle M mit der Oxidationszahl m = 1 sind: Lithium, Natrium und Kalium, bevorzugt Natrium, und für Metalle M mit der Oxidationszahl m = 2 sind: Magnesium und Calcium.

X steht für ein Anion mit der Ladungszahl n (n = 1, 2 oder 3) ausgewählt aus der Gruppe
ClO₄⁻, B(C₆H₅)₄⁻, SbF₆⁻, AsF₆⁻, PF₆⁻, SiF₆²⁻, SO₄²⁻, HSO₄⁻, NO₃⁻, PO₄³⁻, HPO₄²⁻, H₂PO₄⁻, HCO₃⁻, CO₃²⁻.

Bevorzugte Verbindungen der Formel MₙXₘ sind Natriumtetraphenylborat, Kaliumtetraphenylborat, Natriumperchlorat, Kaliumperchlorat, Lithiumperchlorat, Perchlorsäure, Natriumhexafluorantimonat, Natriumhexafluorarsenat, besonders bevorzugt Natriumtetraphenylborat, Natriumperchlorat und Perchlorsäure.

Die Umsetzung zu den 1,2,4-Triazoliumsalzen Ib bzw. IIb erfolgt im allgemeinen in flüssiger Phase, wobei gewöhnlich das 1,2,4-Triazoliumsalz Ia bzw. IIa, gegebenenfalls in einen Lösungsmittel gelöst, vorgelegt und mit der Verbindung MₙXₘ, gegebenenfalls in einem Lösungsmittel gelöst, versetzt wird.

Das molare Verhältnis von Ib zur Verbindung MₙXₘ kann beispielsweise im Bereich von 1:1 bis 1:200, bevorzugt 1:1 bis 1:10, liegen.

Das molare Verhältnis von IIb zur Verbindung MₙXₘ kann beispielsweise im Bereich von 1:2 bis 1:400, bevorzugt 1:2 bis 1:20, liegen.

Die Ausgangsverbindungen Ia bzw. IIa müssen zur Darstellung der Produkte Ib und IIb nicht in reiner Form vorliegen. In den meisten Fällen können die rohen Reaktionsprodukte des erfindungsgemäßen Herstellverfahrens für Ia bzw. IIa eingesetzt werden.

Die Reaktionszeiten liegen gewöhnlich im Bereich von 1 Minute bis 1 Tag.

Die Umsetzung erfolgt in der Regel bei Temperaturen von 0 bis 50°C, bevorzugt 20 bis 30 °C. Sie kann diskontinuierlich oder kontinuierlich, drucklos oder unter Druck, bevorzugt diskontinuierlich und bei Normaldruck, in gängigen Reaktoren, wie zum Beispiel Rührkessel oder Rührbehälterkaskaden, durchgeführt werden.

Bevorzugte Lösungsmittel für diese Reaktion sind Wasser, Alkohole, wie Methanol, Ethanol, Propanol, Isopropanol, Butanol, Ether, wie Tetrahydrofuran, oder Gemische hiervon, bevorzugt Wasser.

Die Reinigung und Isolierung der 1,2,4-Triazoliumsalze Ib und IIb erfolgt in der Regel wie oben für die 1,2,4-Triazoliumsalze Ia und IIa beschrieben.

Die Imidoylverbindungen III sind zum Teil bekannt oder in an sich bekannter Weise, zum Beispiel gemäß 'The chemistry of the carbon-nitrogen double bond, Ed.: S. Patai, Chapter 8: R.J. Morath, Substitution reactions at the azomethine carbon and nitrogen bond, J. Wiley, New York (1970)', 'The chemistry of the carbon-nitrogen double bond, Ed.: S. Patai, Chapter 13:, R. Bonnett, Imidoyl halides, J. Wiley, New York (1970)' und 'H. Ulrich, The chemistry of imidoyl halides, Seiten 56-73, Plenum, New York (1968)' sowie der jeweils dort zitierten Literatur, erhältlich.

Eine Vielzahl von 1-substituierten 1-Formyl-hydrazinen V ist bekannt und zum Beispiel nach den Methoden, wie sie in 'The chemistry of amides, Ed.: S. Patai, Chapter 10: H. Paulsen, D. Stoye, The chemistry of hydrazides, Seite 525ff, J. Wiley, New York (1970)' und dort zitierter Literatur beschrieben wurden, herstellbar.

Die Ausgangsverbindungen III bzw. IV und V müssen zur Darstellung der Produkte Ia und IIa nicht in reiner Form vorliegen. In vielen Fällen können sie direkt nach ihrer Herstellung in ungereinigter Form zur erfindungsgemäßen Herstellung der 1,2,4-Triazoliumsalze Ia und IIa eingesetzt werden.

### Beispiele

### Beispiel 1

### Synthese von 1,3,4-Trimethyl-4H-triazol-1-iumtetraphenylborat

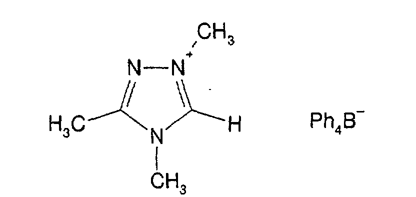

N-Methylacetamid (35,0 g, 479,5 mmol) wurde in 250 ml trockenem Dichlormethan gelöst und auf -78°C abgekühlt. POCl₃ (97,8 g, 677,3 mmol) wurde dann bei dieser Temperatur langsam zugetropft (exotherme Reaktion). Man ließ die Temperatur dann langsam bis auf -10 °C steigen und tropfte bei dieser Temperatur 1-Formyl-1-methylhydrazin (47,0 g, 636,5 mmol) hinzu. Dabei wurde darauf geachtet, daß die Temperatur 0 °C nicht überstieg. Danach wurde Acetanhydrid (116 ml, 1,23 mol) zugetropft und das Reaktionsgemisch 2,5 Stunden am Rückfluß gekocht. Nach dem Abkühlen wurde das Dichlormethan am Rotationsverdampfer bei Raumtemperatur entfernt und der Rückstand (Öl, 197 g) mit einer konzentrierten wässrigen Lösung von Natriumtetraphenylborat versetzt, wobei das Produkt ausfiel. Das Produkt wurde anschließend umgehend abfiltriert (das Produkt wird sonst schmierig und läßt sich nicht mehr filtrieren), mit wenig kaltem Wasser gewaschen und im Vakuum-Trockenschrank bei 45 °C getrocknet. Man erhielt 124,4 g Produkt (Ausbeute ca. 60%) in Form farbloser Kristalle. Zur Reinigung wurde das Produkt in heißem Aceton gelöst und dann mit Diethylether gefällt.
Farblose Kristalle. Smp.: 257 °C.
Elementaranalyse: ber. für C₂₉H₃₀BN₃: 80,74 % C, 7,01 % H, 2,51 % B, 9,74 % N; gef.: 80,5 % C, 6,9 % H, 2,5 % B, 9,6 % N.
MS (ESI+): 112, Trimethyltriazoliumkation; (ESI-): 319, Tetraphenylboratanion.
¹H-NMR (400 MHz, DMSO-d₆): δ (ppm) = 9,7 (1H, s, Triazol-CH), 7,2 (8H, br, o-PhH), 6,9 (8H, m, m-PhH), 6,8 (4H, m, p-PhH), 3,95 (3H, s, 1-CH₃), 3,7 (3H, s, 4-CH₃), 2,45 (3H, s, 3-CH₃).
¹³C-NMR (100 MHz, DMSO-d₆) : δ (ppm) = 163,3 (q, PhC-B, 1J_{C-B}= 49,3 Hz), 153,5 (Triazol-C₃), 143,3 (Triazol-C₅), 135,4 (o-Ph), 125,2 (q, m-Ph, 3J_{C-B}= 2,8 Hz), 121,4 (p-Ph), 37,2 (3-CH₃), 32,5 (4-CH₃), 9,4 (1-CH₃).

### Beispiel 2

### (R)-1,3-Dimethyl-4-(1-phenylethyl)-4H-1,2,4-triazol-1-iumtetraphenylborat

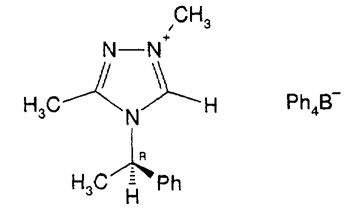

Zu 5,0 g (30 mmol) (R)-N-1-Phenylethylacetamid [L. Duhamel, J.-C. Plaquevent, *Bull. Soc. Chim. Fr.* (1982) *2*, 75-83] in 40 ml trokkenem Methylenchlorid wurden bei Raumtemperatur 6,1 (42 mmol) POCl₃ langsam zugegeben, es wurde noch 30 Min. bei Raumtemperatur gerührt und dann die Lösung auf 0 °C gekühlt. Bei 0-10 °C wurden 2,9 g (40 mmol) 1-Methyl-1-formylhydrazin zugetropft. Es fiel ein weißer Feststoff aus. Nach Zugabe von 10 ml Acetanhydrid wurde die Lösung 3 h zum Rückfluß erhitzt, wobei sich der größte Teil des Feststoffes auflöste. Nach dem Abkühlen schied sich ein unlöslischer, klarer und öliger Feststoff ab, von dem die überstehende Lösung abdekantiert und diese dann am Rotationsverdampfer eingeengt wurde. Es verblieben 20 g eines roten Öls.

2 g des erhaltenen Öls wurden mit einer gesättigten wässrigen Lösung von NaB(C₆H₅)₄ versetzt. Der erhaltene Feststoff wurde abfiltriert und im Hochvakuum getrocknet. Es wurden 1,3 g Produkt erhalten (Ausbeute: 67 %), das laut NMR noch ca. 0,2 Gew.-% (R)-N-1-Phenylethylacetamid enthielt. Zur Reinigung kann das Produkt aus heißem Methanol umkristallisiert werden.
Hellgelbe Kristalle. Smp.: 149 °C.
Elementaranalyse: ber. für C₃₆H₃₆BN₃: 83,03 % C, 6,97 % H, 1,93 % B, 8,07 % N; gef.: 82,92 % C, 7,02% H, 8,03 % N. MS (SIMS+): 202,1 (100,0, C₁₂H₁₆N₃⁺) ; 105,0 (49,3); 98,0 (46,0); (SIMS-): 319,1 (100,0, B(C₆H₅)₄⁻).
¹H-NMR (300 MHz, DMSO-d₆) : δ (ppm) = 10,28 (1H, s, NCH), 7,42 (6H, m, C₆H₅), 7,18 (8H, m, C₆H₅), 6,93 (7H, m, C₆H₅), 6,79 (4H, m, C₆H₅), 5,77 (1H, q, 7,1 Hz, CHCH₃), 3,99 (3H, s, NCH₃), 2,36 (3H, s, CCH₃), 1,84 (3H, d, 7,1 Hz, CHCH₃).
¹³C-NMR (75 MHz, DMSO-d₆): δ (ppm) = 163,26 (q, J = 50 Hz, C₅H₅CB), 152,39, 142,31 (NCH, CCH₃), 138,03, 135,45, 129,05, 128,72, 126,42 (C₆H₅), 125,19, 121,42 (q, J = 3 Hz), 56,71 (CHCH₃), 38,47 (NCH₃), 21,36 (CHCH₃), 10,09 (CCH₃).
IR-Spektrum (KBr-Preßling): ν (cm⁻¹) = 3060, 3000, 2990 (m), 1650 (br w), 1580 (s), 1560, 1480 (m), 1430 (s), 1380, 1150, 770 (m), 740, 700 (s).
Drehwert: [α]_{D}²⁵ = -20 (0,5, MeOH).

### Beispiel 3

### (R)-1,3-Dimethyl-4-(1-phenylethyl)-4H-1,2,4-triazol-1-iumperchlorat

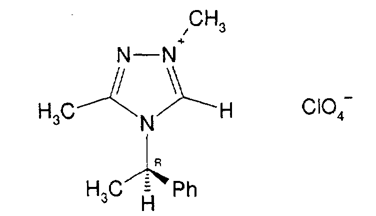

Um das Perchlorat herzustellen wurde zunächst wie oben verfahren. Das rote Öl (2 g) wurde aber unter Rühren mit einer gesättigten wässrigen Lösung von Natriumperchlorat versetzt. Es entstand ein öliger Feststoff, der von der Lösung abgetrennt und mit wenig Ethanol verrieben wurde. Zu der ethanolischen Lösung wurde Diethylether gegeben und es fiel ein hellgelber Feststoff aus, der abfiltriert wurde (0,76 g, 84 % Ausbeute). Zur Reinigung kann das Produkt aus heißem Methanol umkristallisiert werden.
Hellgelbe Kristalle. Smp.: 152-154 °C.
Elementaranalyse: ber. für C₁₂H₁₆ClN₃O₄: 47,77 % C, 5,34 % H, 11,75 % Cl, 13,93 % N, 21,21 % O; gef.: 47,67 % C, 5,38 % H, 14,00 % N. MS (SIMS+): 202,1 (100,0, C₁₂H₁₆N₃⁺); 105,0 (24,1); 98,0 (28,9); (SIMS-): 98,8 (100,0, ClO₄⁻).
¹H-NMR (300 MHz, DMSO-d₆): δ (ppm) = 10,25 (1H, s, NCH), 7,43 (5H, m, C₆H₅), 5,79 (1H, q, 7,1 Hz, CHCH₃), 4,02 (3H, s, NCH₃), 2,37 (3H, s, CCH₃), 1,85 (3H, d, 6,7 Hz, CHCH₃).
¹³C-NMR (75 MHz, DMSO-d₆): δ (ppm) = 152,51, 142,29 (NCH, CCH₃), 138,06, 129,07, 128,72, 126,43 (C₆H₅), 56,72 (CHCH₃), 38,51 (NCH₃), 21,36 (CHCH₃), 10,09 (CCH₃).
IR-Spektrum (KBr-Preßling): ν (cm⁻¹) = 3100, 3060, 2990, 1600, 1560, 1500 (w), 1440, 1390 (m), 1100 (br s), 770, 710 (m), 620 (s) .
Drehwert: [α]_{D}²⁵ = -51 (0,5, MeOH).

### Beispiel 4

### 1,3-Dimethyl-4-((1S,2R)-2-acetoxy-2-phenyl-1-methylethyl)-4H-1,2,4-triazol-1-iumtetraphenylborat

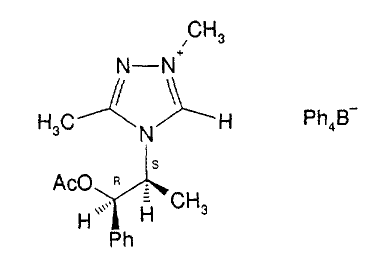

Zu 17,8 g (30 mmol) acetylgeschütztem (1R,2S)-(-)-Norephedrin in 40 ml trockenem Methylenchlorid wurden bei Raumtemperatur 6,1 g (42 mmol) POCl₃ langsam zugegeben. Es wurde noch 30 Min. bei Raumtemperatur gerührt und dann die Lösung auf 0°C gekühlt. Bei 0-10 °C wurden 2,9 g (40 mmol) 1-Methyl-l-formylhydrazin zugetropft und es fiel ein weißer Feststoff aus. Nach Zugabe von 10 ml Acetanhydrid wurde die Lösung für 3 h zum Rückfluß erhitzt. Nach dem Abkühlen schied sich ein unlöslicher, klarer und öliger Feststoff ab, von dem die überstehende Lösung abdekantiert und diese dann am Rotationsverdampfer eingeengt wurde. Es verblieb ein rotes Öl.

Das erhaltene Öl wurde dann mit einer gesättigten wässrigen Lösung von NaB(C₆H₅)₄ versetzt, wobei das Produkt in Form hellgelber Kristalle ausfiel. Der erhaltene Feststoff wurde abfiltriert und im Hochvakuum getrocknet. Es wurden 6,4 g Produkt erhalten (Ausbeute: 41 %). Zur Reinigung kann das Produkt aus heißem Methanol umkristallisiert werden.
Hellgelbe Kristalle. Smp.: 152 °C.
Elementaranalyse: ber. für C₃₉H₄₀BN₃O₂: 78,92 % C, 6,79 % H, 1,82 % B, 7,08 % N, 5,39 % O; gef.: 78,73 % C, 6,76 % H, 7,41 % N. MS (SIMS+): 274 (100,0, M⁺); 177 (58,5); 143 (30,3).
¹H-NMR (300 MHz, DMSO-d₆): δ (ppm) = 1,49 (d, J= 6,72 Hz, 3H, CH₃CHN), 2,17 (s, 3H, COCH₃), 2,48 (s, 3H, N=C-CH₃), 3,98 (s, 3H, NCH₃), 4,96 (q/d, J= 7,05 / 4,37 Hz, 1H, NCH-CH₃), 5,93 (d, J= 4,71 Hz, 1H, PhCHO), 7,28 (m, 2H, Ph), 7,40 (m, 3H, Ph), 10,02 (s, 1H, NCHN).
¹³C-NMR (75 MHz, DMSO-d₆): δ (ppm) = 9,80 (NCCH₃), 14,65 (NCHCH₃), 20,63 (COCH₃), 38,54 (NCH₃), 56,49 (NCHCH₃), 75,13 (PhCHO), 126,17 (Ph), 128,58 (Ph), 128,60 (Ph), 135,42 (Ph), 142,30 (NCCH₃), 152,97 (NCHN), 169,13 (COCH₃).
IR-Spektrum (KBr-Preßling, vom Perchlorat): ν (cm⁻¹) = 3100 (w), 1751 (s), 1655 (m), 1590 (m), 1562 (m), 1455 (m), 1377 (m), 1201 (s), 1097 (br s), 760 (w), 708 (m), 624 (s).
Drehwert: [α]_{D}²⁵ = -19 (0,5, MeOH).

### Beispiel 5

### 1,4-Dimethyl-3-phenyl-4H-1,2,4-triazol-1-iumchlorid

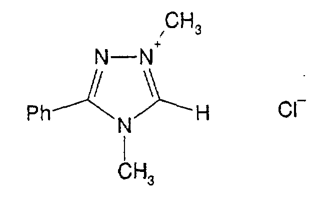

N-Methylbenzimidoylchlorid [v. Braun, Pinkernelle, *Chem. Ber.* (1895), *28*, 1218] (60,0 g, 0,39 mol, frisch destilliert) wurden in 300 ml Dichlormethan gelöst. l-Formyl-l-methylhydrazin (38,5 g, 0,52 mol) wurden dann langsam bei Raumtemperatur zugetropft (exotherme Reaktion). Nach 15 Min. wurden dann 110 g Essigsäureanhydrid zugetropft und das Gemisch 2 Stunden unter Rückfluß erhitzt. Nach dem Abkühlen wurde das ausgefallene Produkt abfiltriert, mit Diethylether gewaschen, getrocknet und aus Ethanol/ Diethylether umkristallisiert. Ausbeute: 51,3 g (58 %) als Monohydrat.
Farblose Kristalle. Smp.: 65 °C.
Elementaranalyse: ber. für C₁₀H₁₄ClN₃O (227,7): 52,75 % C, 6,20 % H, 15,57 % Cl, 18,45 % N, 7,03 % O; gef.: 52,0 % C, 6,2 % H, 15,2 % Cl, 18,0 % N, 9,3 % O.
MS (ESI+): 174 (M⁺); (ESI-): 35, 37 (Cl).
¹H-NMR (300 MHz, CDCl₃), δ (ppm) = 3,85 (s, breit, 2-3H, Wasser), 4,20 (s, 3H, NCH₃), 4,33 (s, 3H, NCH₃), 7,6 (m, 3H, Ph), 7,8 (m, 2H, Ph).
¹³C-NMR (100 MHz, CDCl₃): δ (ppm) = 35,25 (N(4)-CH₃), 39,49 (N(1)-CH₃), 122,50 (q Ph), 129,43 (Ph), 129,58 (Ph), 132,22 (Ph), 145,53 (N=CH), 154,42 (Ph-C=N).
IR-Spektrum (KBr-Preßling): ν (cm⁻¹) = 3035 (w), 2921 (w), 1587 (s), 1575 (s), 1548 (s), 1503 (m), 1452 (s), 1363 (m), 1189 (s), 1083 (m), 1024 (w), 790 (s), 725 (s), 705 (s), 629 (m), 553 (m).

### Beispiel 6

### 4,4'-(1,6-Hexandiyl)-bis[1-methyl-3-phenyl-4H-1,2,4-triazol-1-iumchlorid]

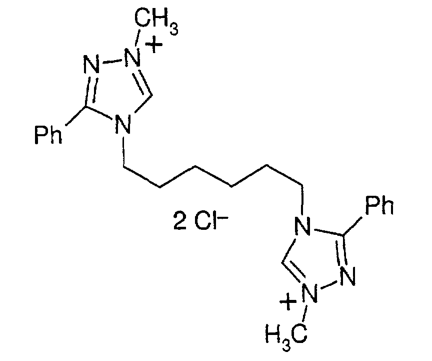

Hexamethylendiamin wurde mit Benzoylchlorid unter Bildung von N,N'-Dibenzoyl-hexamethylendiamin [G. Cirrincione, W. Hinz, R. A. Jones, *J. Chem. Soc. Perkin Trans. 2,* 1984, 1089-1092] umgesetzt, welches dann durch Behandlung mit Thionylchlorid unter Rückfluß über Nacht und anschließendes Entfernen des überschüssigen Thionylchlorids *in vacuo* in das Hexamethylen-N,N'-bisbenzimidoylchlorid überführt wurde. 10 g von diesem wurden in 200 ml abs. Dichlormethan gelöst und die resultierende Lösung filtriert. Die Lösung wurde auf ca. 40 ml eingeengt und auf 0 °C gekühlt. Dann wurde 1-Formyl-1-methylhydrazin (5,4 g) und Pyridin (8,8 g) zugesetzt und 2 h bei RT gerührt, worauf Acetanhydrid (15,6 g) zugegeben und die resultierende Lösung 2 Tage refluxiert wurde. Die erhaltene dunkelbraune Lösung wurde eingeengt und zu einer gesättigten wässrigen Lösung von NaB(C₆H₅)₄ gegeben, wobei ein dunkelbrauner Feststoff entand. Das Rohprodukt wurde nach säulenchromatographischer Reinigung (Kieselgel, CH₂Cl₂/MeOH = 3/1) mit einer Ausbeute von 31 % in Form weißer Kristalle erhalten.
MS (ESI+): 402 (M⁺).
¹H-NMR (300 MHz, DMSO-d₆): δ (ppm) = 1,21 (m, 4H, 2 CH₂), 1,75 (m, 4H, 2 CH₂), 4,20 (s, 6H, 2 NCH₃), 4,27 (t, 6H, J = 7 Hz, 2 NCH₂CH₂), 7,63 - 7,85 (m, 10 H, 2 Ph), 10,95 (s, 1H, NC(H)N).
¹³C-NMR (100 MHz, DMSO-d₆) : δ (ppm) = 25,04 (2 CH₂), 28,48 (2 CH₂), 39,37 (2 CH₃), 47,53 (2 CH₂) , 123,43 (ipso-Cₐᵣ), 129,82 (Cₐᵣ), 129,88 (Cₐᵣ), 132,51 (Cₐᵣ), 144,67 (NC(H)N), 153,88 (C=N).

### Beispiel 7

### 4-[2-(p-Nitrobenzoylamino)-cyclohexyl]-1-methyl-3-(p-nitrophenyl) -4H-1,2,4-triazol-1-iumtetraphenylborat

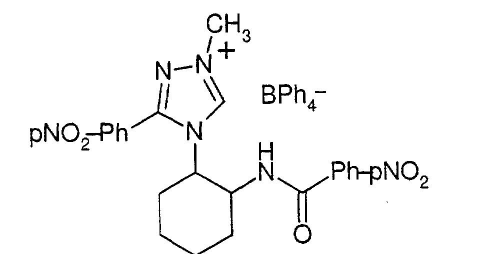

(*rac*.)-*trans-Bis*-(*p*-nitrobenzoyl)-cyclohexandiamin (2 g) wurden in 10 ml Thionylchlorid 2 Tage unter Rückfluß erhitzt. Die Lösung wurde im Hochvakuum eingeengt, der Rückstand in abs. Dichlormethan gelöst und die resultierende Lösung filtriert. Die Lösung wurde auf ca. 20 ml eingeengt und auf 0°C gekühlt. Dann wurde 1-Formyl-1-methylhydrazin (1,1 g) zugesetzt und 2 h bei RT gerührt, worauf Acetanhydrid (3 ml) zugegeben und die resultierende Lösung 2 Tage refluxiert wurde. Die erhaltene dunkelbraune Lösung wurde eingeengt und zu einer gesättigten wässrigen Lösung von NaB(C₆H₅)₄ gegeben, wobei ein dunkelbrauner Feststoff entstand, der durch Waschen mit Methanol gereinigt werden kann. Nach säulenchromatographischer Reinigung (Kieselgel, CH₂Cl₂/MeOH = 3/1) wurde das 1,2,4-Triazoliumsalz in Form weißer Kristalle mit einer Ausbeute von 27 % (1 g) erhalten.
Farblose Kristalle. Smp.: >220 °C.
Elementaranalyse: ber. für C₄₆H₄₃BN₆O₅ (770,7): 71,69 % C, 5,62 % H, 10,90 % N; gef.: 72,1 % C, 5,3 % H, 10,5 % N.
MS (SIMS+): 451,2 (M⁺) ; (SIMS-): 318,9 (B(C₆H₅)₄⁻).
¹H-NMR (300 MHz, CDCl₃): δ (ppm) = 1,40 (m, 1H, CH₂), 1,52 (m, 1H, CH₂), 1,78 (m, 1H, CH₂), 1,85 (m, 2H, CH₂), 2,10 (m, 1H, CH₂), 2,30 (m, 1H, CH₂), 4,17 (s, 3H, NCH₃), 4,23 (m, 2H, CH), 6,78 (m,
4H, p-PhB), 6,92 (m, 8H, o/m-PhB), 7,18 (m, 8H, o/m-PhB), 7,71 (d, 2H, J = 6,3 Hz, COPh-pNO₂), 7,79 (d, 2H, J = 7,1 Hz, COPhpNO₂), 8,31 (d, 2H, J = 6,6 Hz, COPh-pNO₂), 8,40 (d, 2H, J = 6,6 Hz, COPh-pNO₂), 8,58 (m, 1H, NH), 10,87 (s, 1H, NC(H)N).
¹³C-NMR (75 MHz, CDCl₃): δ (ppm) = 23,87, 30,66, 31,11 (CH₂), 39,15 (CH₃), 53,26 (CH), 62,25 (CH), 121,42 (p-PhB), 123,62 (Ph-NO₂) , 124,52 (Ph-NO₂), 125,23 (o-PhB), 128,49 (Ph-NO₂), 131,10 (Ph-NO₂), 135,46 (m-PhB), 138,43, 145,58, 150,17, 150,40, 153,36 (Cᵢₚₛₒ, NC(H)N), 164,26 (q, J = 49 Hz, PhB-Cᵢₚₛₒ), 163,48, 164,54 (CONH, NO₂-PhC=N).
IR-Spektrum (KBr-Preßling): ν (cm⁻¹) = 3054 (m), 2999 (w), 2931 (m), 2861 (m), 1671 (m), 1643 (m), 1601 (m), 1567 (m), 1526 (s), 1481 (m), 1450 (m), 1349 (s), 863 (m), 829 (m), 735 (m), 708 (s).

## Patentansprüche

1. Verfahren zur Herstellung von 1,3,4-trisubstituierten 1,2,4-Triazoliumsalzen der Formeln Ia bzw. IIa 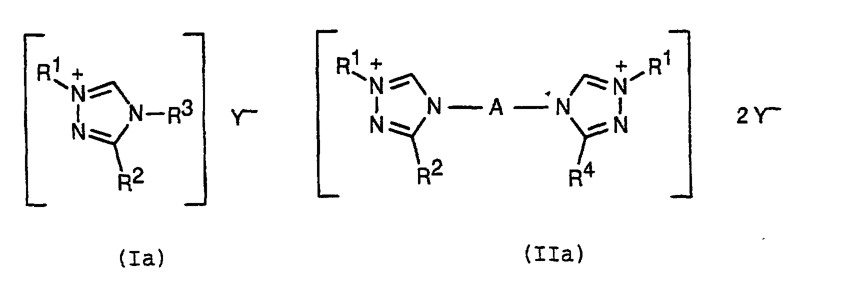 in denen die Substituenten und Variablen unabhängig voneinander
R¹ C₁₋₃₀-Alkyl, C₃₋₁₂-Cycloalkyl, C₆₋₂₀-Aryl, C₇₋₂₀-Arylalkyl, C₃₋₁₅-Heteroaryl,
R², R³, R⁴ C₁₋₃₀-Alkyl, C₂₋₃₀-Alkenyl, C₃₋₁₂-Cycloalkyl, C₅₋₁₂-Cycloalkenyl, C₆₋₂₀-Aryl, C₇₋₂₀-Arylalkyl, C₃₋₁₅-Heteroaryl, oder R² und R³ gemeinsam eine gesättigte oder ungesättigte C₃₋₆-Alkylkette, in der bis zu 2 C-Atome unabhängig voneinander durch -N= (Imino), O, S, NR⁵ oder CHR⁶ substituiert sein können, oder R² und R⁴ gemeinsam (CH₂)ₐ-E-(CH₂)_{b},
wobei der Rest R¹ Substituenten, ausgewählt aus der Gruppe C₁₋₃₀-Alkyl und/oder C₁₋₃₀-Alkoxy, und die Reste R² bis R⁴ Substituenten, ausgewählt aus der Gruppe Halogene, C₁₋₃₀-Alkyl, C₁₋₃₀-Alkoxy, C₁₋₃₀-Alkanoyloxy, C₆₋₃₀-Aryloxy, C₂₋₂₀-Dialkylamino, Benzoylamino, p-Nitro-benzoylamino und/oder Nitro, tragen können,
A (CH₂)_{c}-Bₑ-(CH₂)_{d}
E CH₂, CHR⁵, O, S, NR⁶,
B CH₂, CHR⁷, O, S, NR⁸,
R⁵, R⁶, R⁷, R⁸ C₁₋₄-Alkyl, C₇₋₂₀-Alkylaryl, C₇₋₂₀-Arylalkyl,
a,c eine ganze Zahl von 1 bis 3,
b,d eine ganze Zahl von 1 bis 30,
e 0 oder 1,
Y⁻ ein Anion, ausgewählt aus der Gruppe der Halogenide, Carboxylate der Formeln R⁹CO₂⁻, HCO₂⁻, CF₃CO₂⁻ und p-NO₂-C₆H₄-CO₂⁻, Carbonate der Formel R⁹OCO₂⁻, Sulfate der Formel R⁹OSO₃⁻, Sulfonate der Formeln R⁹SO₃⁻, CF₃SO₃⁻ und p-NO₂-C₆H₄-SO₃⁻, Sulfinate der Formel R⁹SO₂⁻, Imidazolide und Cyanid und
R⁹ ein aliphatischer, cycloaliphatischer, arylaliphatischer, aromatischer oder alkylaromatischer Rest
bedeuten, **dadurch gekennzeichnet, daß** man eine Imidoylverbindung der Formel III bzw. eine Bis-imidoylverbindung der Formel IV 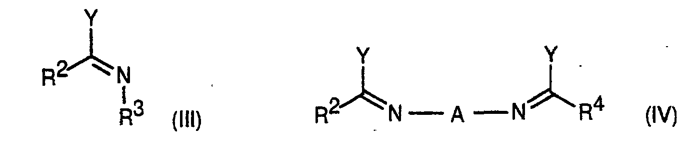 mit einem N-Formyl-hydrazin der Formel V 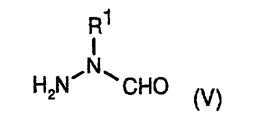 bei Temperaturen von -78° bis 130°C umsetzt.

2. Verfahren nach Anspruch 1, **dadurch gekennzeichnet, daß** man eine Imidoylverbindung der Formel III 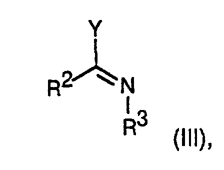 in der
R² C₁₋₃₀-Alkyl, C₆₋₂₀-Aryl,
R³ C₁₋₃₀-Alkyl, C₃₋₁₂-Cycloalkyl, C₆₋₂₀-Aryl, C₇₋₂₀-Arylalkyl,
wobei die Reste R² und R³ Substituenten, ausgewählt aus der Gruppe Cl, C₁₋₃₀-Alkyl, C₁₋₃₀-Alkoxy, C₁₋₃₀-Alkanoyloxy, C₆₋₃₀-Aryloxyl, C₂₋₂₀-Dialkylamino, Benzoylamino, p-Nitro-benzoylamino, Nitro, tragen können, und
Y ein Substituent, ausgewählt aus der Gruppe F-, Cl-, Br-, I-, CH₃OCO₂-, C₂H₅OCO₂-, C₆H₅CH₂OCO₂-, HCO₂-, CH₃CO₂-, C₂H₅CO₂-, CF₃CO₂-, p-NO₂-C₆H₄-CO₂-, CH₃OSO₃-, CH₃SO₃-, CF₃SO₃-, C₆H₅-SO₃-, p-CH₃-C₆H₄-SO₃-, p-NO₂-C₆H₄-SO₃-, CH₃SO₂-, C₆H₅-SO₂-, NC- und Imidazol-1-yl,
bedeuten,
mit einem N-Formyl-hydrazin der Formel V 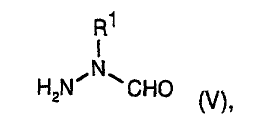 in der
R¹ C₁₋₃₀-Alkyl, C₆₋₂₀-Aryl,
bedeutet, umsetzt.

3. Verfahren nach Anspruch 1, **dadurch gekennzeichnet, daß** man eine Bis-imidoylverbindung der Formel IV 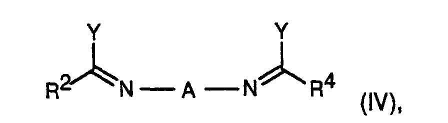 in der
R², R⁴ C₁₋₃₀-Alkyl, C₃₋₁₂-Cycloalkyl, C₆₋₂₀-Aryl, C₇₋₂₀-Arylalkyl,
wobei die Reste R² und R⁴ Substituenten, ausgewählt aus der Gruppe Cl, C₁₋₃₀-Alkyl, C₁₋₃₀-Alkoxy, C₁₋₃₀-Alkanoyloxy, C₆₋₃₀-Aryloxy, C₂₋₂₀-Dialkylamino, Benzoylamino, p-Nitro-benzoylamino, Nitro, tragen können,
bedeuten,
mit einem N-Formyl-hydrazin der Formel V gemäß Anspruch 1, in der
R¹ C₁₋₃₀-Alkyl, C₆₋₂₀-Aryl,
bedeutet, umsetzt.

4. Verfahren nach den Ansprüchen 1 bis 3, dadurch gekennnzeichnet, daß man die Umsetzung bei Temperaturen von -40 bis +70°C durchführt.

5. Verfahren nach den Ansprüchen 1 bis 3, dadurch gekennnzeichnet, daß man die Umsetzung in Gegenwart einer Verbindung, ausgewählt aus der Gruppe der Carbonsäureanhydride oder Carbodiimide, durchführt.

6. Verfahren nach den Ansprüchen 1 bis 3, dadurch gekennnzeichnet, daß man die Umsetzung in Gegenwart einer Base, ausgewählt aus der Gruppe der tertiären Amine oder aromatischen N-Heterocyclen, durchführt.

7. Verfahren zur Herstellung von 1,3,4-trisubstituierten 1,2,4-Triazoliumsalzen der Formeln Ib bzw. IIb 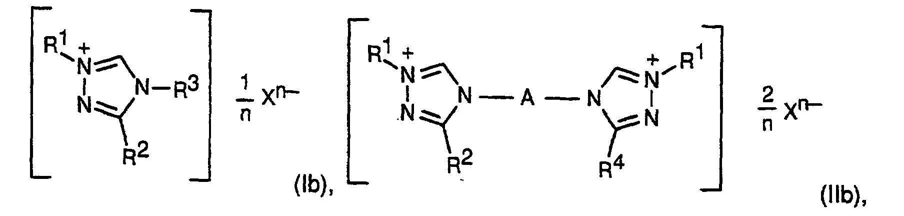 in denen
Xⁿ⁻ ein Anion mit der Ladungszahl n, ausgewählt aus der Gruppe ClO₄⁻, B(C₆H₅)₄⁻, SbF₆⁻, AsF₆⁻, PF₆⁻, SiF₆²⁻, SO₄²⁻, HSO₄⁻, NO₃⁻, PO₄³⁻, HPO₄²⁻, H₂PO₄⁻, HCO₃⁻, CO₃²⁻,
n 1, 2 oder 3
bedeuten und R¹, R², R³, R⁴ und A die in Anspruch 1 angegebene Bedeutung haben,
**dadurch gekennzeichnet, daß** man 1,2,4-Triazoliumsalze der Formeln Ia bzw. IIa nach einem Verfahren gemäß Anspruch 1 herstellt und anschließend mit einer Verbindung der Formel MₙXₘ,
wobei M für Wasserstoff mit-der Oxidationszahl m = 1 oder für ein Metall der ersten oder zweiten Gruppe des Periodensystems mit der Oxidationszahl m steht,
umsetzt.

8. 1,3,4-trisubstituierte 1,2,4-Triazoliumsalze der Formel IIa 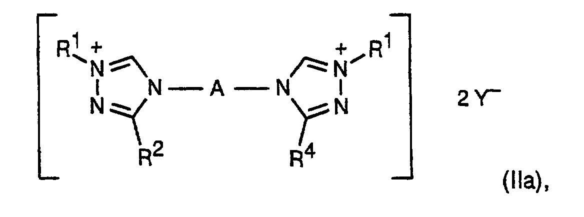 in der R¹, R², R⁴, A und Y die in Anspruch 1 angegebenen Bedeutungen haben.

9. 1,3,4-trisubstituierte 1,2,4-Triazoliumsalze der Formel IIb 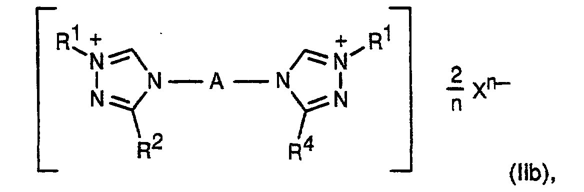 in der R¹, R², R⁴, A die in Anspruch 1 angegebenen Bedeutungen und X die in Anspruch 7 angegebene Bedeutung haben.

## Claims

1. A process for the preparation of 1,3,4-trisubstituted 1,2,4-triazolium salts of the formulae Ia and IIa 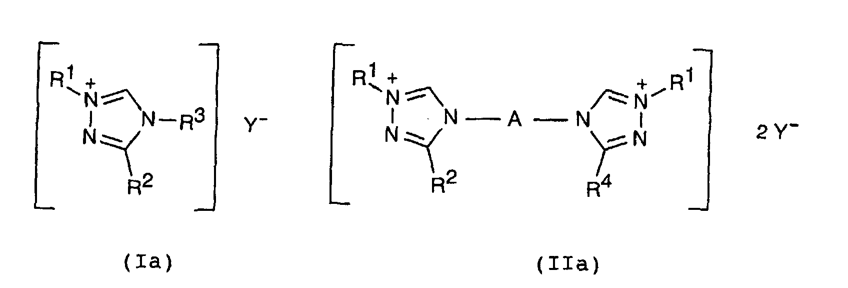 in which independently of one another
R¹ is C₁₋₃₀-alkyl, C₃₋₁₂-cycloalkyl, C₆₋₂₀-aryl, C₇₋₂₀-arylalkyl, C₃₋₁₅-heteroaryl,
R², R³ and R⁴ are C₁₋₃₀-alkyl, C₂₋₃₀-alkenyl, C₃₋₁₂-cycloalkyl, C₅₋₁₂-cycloalkenyl, C₆₋₂₀-aryl, C₇₋₂₀-arylalkyl, C₃₋₁₅-heteroaryl,
or R² and R³ together are a saturated or unsaturated C₃₋₆-alkyl chain, in which up to 2 C atoms can be substituted independently of one another by -N= (imino), O, S, NR⁵ or CHR⁶,
or R² and R⁴ together are (CH₂)ₐ-E-(CH₂)_{b},
where the radical R¹ can carry substituents selected from the group consisting of C₁₋₃₀-alkyl and/or C₁₋₃₀-alkoxy, and the radicals R² to R⁴ can carry substituents selected from the group consisting of halogens, C₁₋₃₀-alkyl, C₁₋₃₀-alkoxy, C₁₋₃₀-alkanoyloxy, C₆₋₃₀-aryloxy, C₂₋₂₀-dialkylamino, benzoylamino, p-nitro-benzoylamino and/or nitro,
A is (CH₂)_{c}-Bₑ-(CH₂)_{d}
E is CH₂, CHR⁵, O, S, NR⁶,
B is CH₂, CHR⁷, O, S, NR⁸,
R⁵, R⁶, R⁷ and R⁸ are C₁₋₄-alkyl, C₇₋₂₀-alkylaryl, C₇₋₂₀-arylalkyl,
a and c are an integer from 1 to 3,
b and d are an integer from 1 to 30,
e is 0 or 1,
Y⁻ is an anion selected from the group consisting of the halides, carboxylates of the formulae R⁹CO₂⁻, HCO₂-, CF₃CO₂- and p-NO₂-C₆H₄-CO₂-, carbonates of the formula R⁹OCO₂-, sulfates of the formulae R⁹OSO₃-, CF₃SO₃- and p-NO₂-C₆H₄-SO₃-, sulfonates of the formula R⁹SO₃⁻, sulfinates of the formula R⁹SO₂⁻, imidazolides and cyanide and
R⁹ is an aliphatic, cycloaliphatic, arylaliphatic, aromatic or alkylaromatic radical
which comprises reacting an imidoyl compound of the formula III or a bis-imidoyl compound of the formula IV 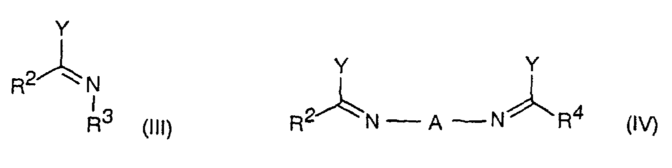 with an N-formylhydrazine of the formula V 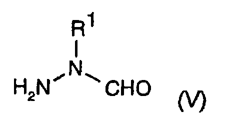 at temperatures from -78° to 130°C.

2. A process as claimed in claim 1, wherein an imidoyl compound of the formula III 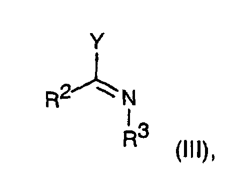 in which
R² is C₁₋₃₀-alkyl, C₆₋₂₀-aryl,
R³ is C₁₋₃₀-alkyl, C₃₋₁₂-cycloalkyl, C₆₋₂₀-aryl, C₇₋₂₀-arylalkyl,
where the radicals R² and R³ can carry substituents selected from the group consisting of Cl, C₁₋₃₀-alkyl, C₁₋₃₀-alkoxy, C₁₋₃₀-alkanoyloxy, C₆₋₃₀-aryloxy, C₂₋₂₀-dialkylamino, benzoylamino, p-nitro-benzoylamino, nitro,
and
Y is a substituent selected from the group consisting of F-, Cl-, Br-, I-, CH₃OCO₂-, C₂H₅OCO₂-, C₆H₅CH₂OCO₂-, HCO₂-, CH₃CO₂-, C₂H₅CO₂-, CF₃CO₂-, p-NO₂-C₆H₄-CO₂-, CH₃OSO₃-, CH₃SO₃-, CF₃SO₃-, C₆H₅-SO₃-, p-CH₃-C₆H₄-SO₃-, p-NO₂-C₆H₄-SO₃-, CH₃SO₂-, C₆H₅-SO₂-, NC- and imidazol-1-yl,
is reacted with an N-formylhydrazine of the formula V 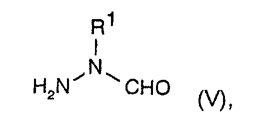 where
R¹ is C₁₋₃₀-alkyl, C₆₋₂₀-aryl.

3. A process as claimed in claim 1, wherein a bis-imidoyl compound of the formula IV 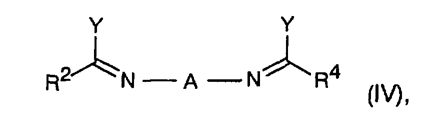 where
R² and R⁴ are C₁₋₃₀-alkyl, C₃₋₁₂-cycloalkyl, C₆₋₂₀-aryl, C₇₋₂₀-arylalkyl,
where the radicals R² and R⁴ can carry substituents selected from the group consisting of Cl, C₁₋₃₀-alkyl, C₁₋₃₀-alkoxy, C₁₋₃₀-alkanoyloxy, C₆₋₃₀-aryloxy, C₂₋₂₀-dialkylamino, benzoylamino, p-nitro-benzoylamino, nitro
is reacted with an N-formylhydrazine of the formula V as claimed in claim 1, where
R¹ is C₁₋₃₀-alkyl, C₆₋₂₀-aryl.

4. A process as claimed in claims 1 to 3, wherein the reaction is carried out at temperatures from -40 to +70°C.

5. A process as claimed in claims 1 to 3, wherein the reaction is carried out in the presence of a compound selected from the group consisting of the carboxylic anhydrides and carbodiimides.

6. A process as claimed in claims 1 to 3, wherein the reaction is carried out in the presence of a base selected from the group consisting of the tertiary amines and aromatic N-heterocycles.

7. A process for the preparation of 1,3,4-trisubstituted 1,2,4-triazolium salts of the formulae Ib and IIb 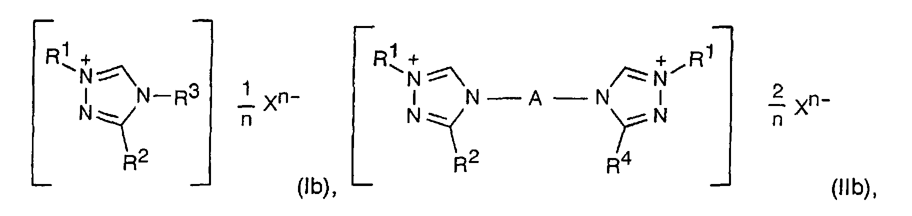 in which
Xⁿ⁻ is an anion having the charge number n, selected from the group consisting of ClO₄⁻, B(C₆H₅)₄⁻, SbF₆⁻, AsF₆⁻, PF₆⁻, SiF₆²⁻, SO₄²⁻, HSO₄⁻, NO₃⁻, PO₄³⁻, HPO₄²⁻, H₂PO₄-, HCO₃⁻, CO₃²⁻,
n is 1, 2 or 3
and R¹, R², R³, R⁴ and A have the meanings indicated in claim 1,
which comprises preparing 1,2,4-triazolium salts of the formulae Ia and IIa by a process as claimed in claim 1, and subsequently reacting them with a compound of the formula MₙXₘ,
where M is hydrogen having the oxidation number m = 1 or is a metal of the first or second group of Periodic Table having the oxidation number m.

8. A 1,3,4-trisubstituted 1,2,4-triazolium salt of the formula IIa 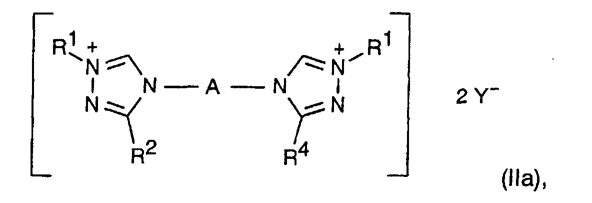 where R¹, R², R⁴, A and Y have the meanings indicated in claim 1.

9. A 1,3,4-trisubstituted 1,2,4-triazolium salt of the formula IIb 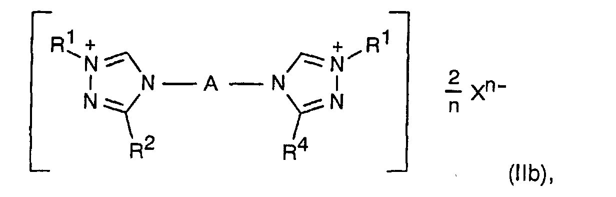 where R¹, R², R⁴ and A have the meanings indicated in claim 1 and X has the meaning indicated in claim 7.

## Revendications

1. Procédé de préparation de sels de 1,2,4-triazolium 1,3,4-trisubstitués des formules la ou lla 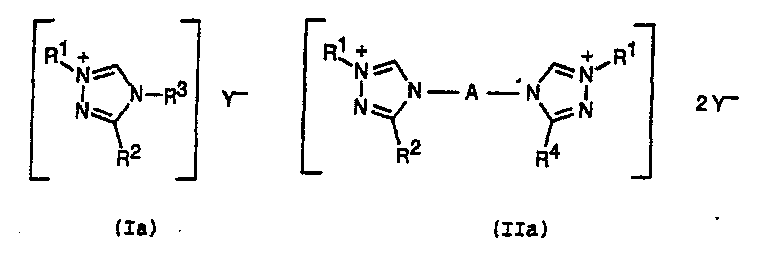 dans lesquelles les substituants et les variables représentent indépendamment les uns des autres
R¹ un radical alkyle en C₁ à ₃₀, cycloalkyle en C₃ à C₁₂, aryle en C₆ à C₂₀, arylalkyle en C₇ à C₂₀, hétéroaryle en C₃ à C₁₅,
R², R³, R⁴ un radical alkyle en C₁ à C₃₀, alcényle en C₂ à C₃₀, cycloalkyle en C₃ à C₁₂, cycloalcényle en C₅ à C₁₂, aryle en C₆ à C₂₀, arylalkyle en C₇ à C₂₀, hétéroaryle en C₃ à C₁₅, ou bien R² et R³ représentent ensemble une chaîne alkyle saturée ou insaturée en C₃ à C₆, dans laquelle jusqu'à 2 atomes de carbone peuvent être, indépendamment l'un de l'autre, substitués par -N= (imino), O, S, NR⁵ ou CHR⁶,
ou bien R² et R⁴ représentent ensemble (CH₂)ₐ-E-(CH₂)_{b},
où les restes R¹ peuvent porter des substituants choisis dans le groupe formé par des radicaux alkyle en C₁ à C₃₀ et/ou alcoxy en C₁ à C₃₀, et les restes R² à R⁴ des substituants choisis dans le groupe formé par les halogènes, des radicaux alkyle en C₁ à C₃₀, alcoxy en C₁ à C₃₀, alcanoyloxy en C₁ à C₃₀, aryloxy en C₆ à C₃₀, dialkylamino en C₂ à C₂₀,benzoylamino, p-nitro-benzoylamino et/ou nitro,
A représente (CH₂)_{c}-Bₑ-(CH₂)_{d}
E représente CH₂, CHR⁵, O, S, NR⁶,
B représente CH₂, CHR⁷, O, S, NR⁸,
R⁵, R⁶, R⁷, R⁸ représentent des radicaux alkyle en C₁ à C₄, alkylaryle en C₇ à C₂₀, arylalkyle en C₇ à C₂₀,
a, c sont des nombres entiers de 1 à 3,
b, d sont des nombres entiers de 1 à 30,
e a une valeur de 0 ou 1,
Y- représente un anion choisi dans le groupe formé par les halogénures, des carboxylates de formule R⁹CO₂-, HCO₂-, CF₃CO₂- et p-NO₂-C₆H₄-CO₂⁻, des carbonates de formule R⁹OCO₂-, des sulfates de formule R⁹SO₃-, des sulfonates de formule R⁹SO₃-, CF₃SO₃- et p-NO₂-C₆H₄-SO₃-, des sulfinates de formule R⁹SO₂-, des imidazolides et des cyanures et
R⁹ représente un reste aliphatique, cycloaliphatique, arylaliphatique, aromatique ou alkylaromatique,
**caractérisé en ce que** l'on fait réagir un composé d'imidoyle de la formule III ou un composé de bis-imidoyle de la formule IV 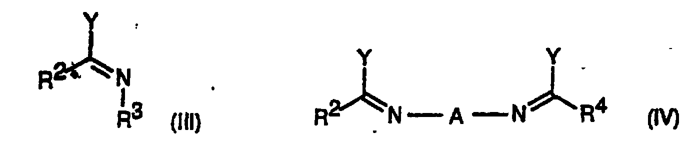 avec une N-formylhydrazine de la formule V 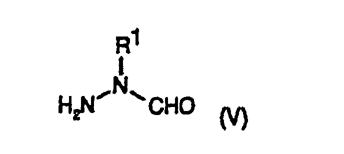 à des températures de -78°C à 130°C.

2. Procédé selon la revendication 1, **caractérisé en ce que** l'on fait réagir un composé d'imidoyle de la formule III 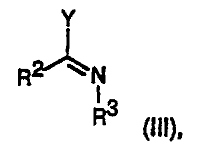 dans laquelle
R² représente un radical alkyle en C₁ à C₃₀, aryle en C₆ à C₂₀,
R³ représente un radical alkyle en C₁ à C₃₀, cycloalkyle en C₃ à C₁₂, aryle en C₆ à C₂₀, arylalkyle en C₇ à C₂₀,
où les restes R² et R³ peuvent porter des substituants choisis dans le groupe formé par CI, ou des radicaux alkyle en C₁ à C₃₀, alcoxy en C₁ à C₃₀, alcanoyloxy en C₁ à C₃₀, aryloxy en C₆ à C₃₀, dialkylamino en C₂ à C₂₀, benzoylamino, p-nitro-benzoylamino, nitro,
et
Y représente un substituant choisi dans le groupe formé par F-, Cl-, Br-, I-, CH₃OCO₂-, C₂H₅OCO₂-, C₆H₅CH₂OCO₂-, HCO₂-, CH₃CO₂-, C₂H₅CO₂-, CF₃CO₂-, p-NO₂-C₆H₄-CO₂-, CH₃OSO₃-, CH₃SO₃-, CF₃CO₃-, C₆H₅SO₃-, p-CH₃-C₆H₄-SO₃-, p-NO₂-C₆H₄-SO₃-, CH₃SO₂-, C₆H₅-SO₂-, NC- et imidazole-1-yle,
avec une N-formylhydrazine de la formule V 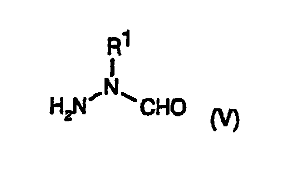 dans laquelle
R¹ représente un radical alkyle en C₁ à C₃₀ ou aryle en C₆ à C₂₀.

3. Procédé selon la revendication 1, **caractérisé en ce que** l'on fait réagir un composé de bis-imidoyle de la formule IV 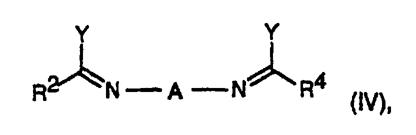 dans laquelle
R², R⁴ représentent des radicaux alkyle en C₁ à C₃₀, cycloalkyle en C₃ à C₁₂, aryle en C₆ à C₂₀, arylalkyle en C₇ à C₂₀,
les restes R² et R⁴ pouvant porter des substituants choisis dans le groupe formé par CI et des radicaux alkyle en C₁ à C₃₀, alcoxy en C₁ à C₃₀, alcanoyloxy en C₁ à C₃₀, aryloxy en C₆ à C₃₀, dialkylamino en C₂ à C₂₀, benzoylamino, p-nitrobenzoylamino, nitro, avec une N-formylhydrazine de la formule V selon la revendication 1, dans laquelle
R¹ représente un radical alkyle en C₁ à C₃₀, aryle en C₆ à C₂₀.

4. Procédé selon les revendications 1 à 3, **caractérisé en ce que** l'on entreprend la réaction à des températures de -40 à +70°C.

5. Procédé selon les revendications 1 à 3, **caractérisé en ce que** l'on entreprend la réaction en présence d'un composé choisi dans le groupe formé par les anhydrides d'acides carboxyliques ou des carbodiimides.

6. Procédé selon les revendications 1 à 3, **caractérisé en ce que** l'on entreprend la réaction en présence d'une base choisie dans le groupe formé par des amines tertiaires ou des N-hétérocycles aromatiques.

7. Procédé de préparation de sels de 1,2,4-triazolium 1,3,4-trisubstitués des formules Ib et IIb 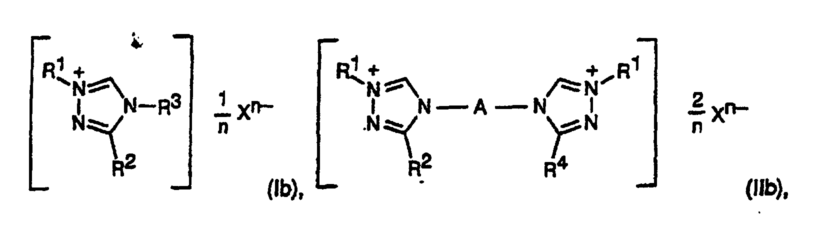 où
Xⁿ⁻ représente un anion du nombre de charge n, choisi dans le groupe formé par ClO₄⁻, B(C₆H₅)₄⁻, SbF₆⁻, AsF₆⁻, PF₆⁻, SiF₆⁻, SO₄²⁻, HSO₄⁻, NO₃⁻, PO₄³⁻, HPO₄²⁻, H₂PO₄⁻, HCO₃⁻, CO₃²⁻,
n a une valeur de 1, 2 ou 3,
et R¹, R², R³, R⁴ et A ont les significations indiquées dans la revendication 1,
**caractérisé en ce que** l'on prépare des sels de 1,2,5-triazolium des formules Ia ou IIa par un procédé selon la revendication 1 et qu'on le fait réagir ensuite avec un composé de formule MₙXₘ,
où M représente de l'hydrogène de l'étage d'oxydation m=1 ou un métal du premier ou du deuxième groupe du système périodique présentant l'étage d'oxydation m.

8. Sels de 1,2,4-triazolium 1,3,4-trisubstitués de formule IIa 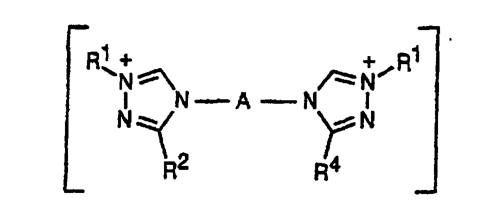 dans laquelle R¹, R², R⁴, A et Y ont les significations indiquées dans la revendication 1.

9. Sels de 1,2,4-triazolium 1,3,4-trisubstitués de formule llb 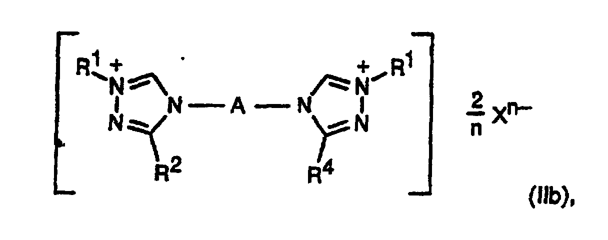 dans laquelle R¹, R², R⁴, A ont les significations indiquées dans la revendication 1 et X la signification indiquée dans la revendication 7.
